# FASCICULE DE BREVET EUROPEEN

(11) **EP 1 616 031 B1**
(45) Date de publication et mention de la délivrance du brevet: **26.11.2008**
(21) Numéro de dépôt: 04742541.8
(22) Date de dépôt: 19.04.2004
(51) Int. Cl.: C12Q 1/68

(54) **PROCEDE DE PREPARATION DE FRAGMENTS D'ADN PAR FRAGMENTATION SELECTIVE D'ACIDES NUCLEIQUES ET SES APPLICATIONS**
VERFAHREN ZUR HERSTELLUNG VON DNA-FRAGMENTEN DURCH SELEKTIVE FRAGMENTIERUNG VON NUKLEINSÄUREN SOWIE ANWENDUNGEN DAVON
METHOD OF PREPARING DNA FRAGMENTS BY SELECTIVE FRAGMENTATION OF NUCLEIC ACIDS AND APPLICATIONS THEREOF

(30) Priorité: 18.04.2003 FR 0304893
(43) Date de publication de la demande: 18.01.2006
(73) Titulaire: COMMISSARIAT A L'ENERGIE ATOMIQUE, 75015 Paris (FR); Université Joseph Fourier, 38041 Grenoble Cédex 9 (FR); CENTRE NATIONAL DE LA RECHERCHE SCIENTIFIQUE, 75794 Paris Cedex 16 (FR)
(72) Inventeur: BRACHET, Anne-Gaelle, F-38260 NANTOIN (FR); RIZO, Philippe, F-38700 LA TRONCHE (FR); TABERLET, Pierre, F-38660 LA TERRASSE (FR); TEXIER-NOGUES, Isabelle, F-38000 Grenoble (FR)
(74) Mandataire: Vialle-Presles, Marie José
(86) Numéro de dépôt international: PCT/FR2004/000963
(87) Numéro de publication internationale: WO 2004/094662

(56) Documents cités:
- EP-A- 0 735 144
- DUNN JOHN J ET AL: "Genomic signature tags (GSTs): A system for profiling genomic DNA." GENOME RESEARCH, vol. 12, no. 11, novembre 2002 (2002-11), pages 1756-1765, XP002304617 ISSN: 1088-9051
- SPINELLA D G ET AL: "Tandem arrayed ligation of expressed sequence tags (TALEST): a new method for generating global gene expression profiles." NUCLEIC ACIDS RESEARCH. 15 SEP 1999, vol. 27, no. 18, 15 septembre 1999 (1999-09-15), pages e22 I-VIII, XP002304618 ISSN: 1362-4962 -& WO 00/53806 A (CHUGAI PHARMACEUTICAL CO LTD) 14 septembre 2000 (2000-09-14)
- VELCULESCU V E ET AL: "SERIAL ANALYSIS OF GENE EXPRESSION" SCIENCE, AMERICAN ASSOCIATION FOR THE ADVANCEMENT OF SCIENCE,, US, vol. 270, no. 5235, 20 octobre 1995 (1995-10-20), pages 484-487, XP001024449 ISSN: 0036-8075
- ZHELEZNAYA L A ET AL: "A METHOD FOR SELECTIVE PCR-AMPLIFICATION OF GENOMIC DNA FRAGMENTS (SAGF METHOD)" BIOCHEMISTRY, CONSULTANTS BUREAU, NEW YORK, NY, US, vol. 60, no. 9, 1995, pages 1037-1043, XP008008712 ISSN: 0006-2979
- YAMAMOTO M ET AL: "Use of serial analysis of gene expression (SAGE) technology" JOURNAL OF IMMUNOLOGICAL METHODS, ELSEVIER SCIENCE PUBLISHERS B.V.,AMSTERDAM, NL, vol. 250, no. 1-2, 1 avril 2001 (2001-04-01), pages 45-66, XP004230694 ISSN: 0022-1759

### SEQUENCE LISTING

<110> commissariat à l'Energie Atomique
   Université Joseph Fourier
   Centre National de la Recherche Scientifique
   BRACHET, Anne-Gaëlle
   RIZO, Philippe
   TABERLET, Pierre
<120> Procédé de préparation de fragments d'ADN par fragmentation sélective des acides nucléiques et ses applications
<130> 263EXT94
<160> 13
<170> Patent In version 3.1
<210> 1
   <211> 15
   <212> DNA
   <213> artificial sequence
<220>
   <223> brin A de l'adaptateur AA'
   <400> 1
   ggaagcctag ctgga 15
<210> 2
   <211> 16
   <212> DNA
   <213> artificial sequence
<220>
   <223> brin A de l'adaptateur AA'
   <400> 2
   ggaagcctag ctggac 16
<210> 3
   <211> 20
   <212> DNA
   <213> artificial sequence
<220>
   <223> brin A' de l'adaptateur AA'
   <220>
   <221> misc_feature
   <222> (1)..(1)
   <223> phosphate
<400> 3
   aattctccag ctaggcttcc 20
<210> 4
   <211> 14
   <212> DNA
   <213> artificial sequence
<220>
   <223> brin B de l'adapatateur BB'
   <220>
   <221> misc_feature
   <222> (1)..(1)
   <223> phosphate
<400> 4
   ggtgagcact catc 14
<210> 5
   <211> 16
   <212> DNA
   <213> artificial sequence
<220>
   <223> brin B' de l'adaptateur BB'
<400> 5
   gatgagtgct gacctt 16
<210> 6
   <211> 16
   <212> DNA
   <213> artificial sequence
<220>
   <223> amorce sens
<400> 6
   ccttcggatc gacctg 16
<210> 7
   <211> 16
   <212> DNA
   <213> artificial sequence
<220>
   <223> amorce anti-sens
<400> 7
   ctactcacga gtggaa 16
<210> 8
   <211> 16
   <212> DNA
   <213> artificial sequence
<220>
   <223> brin A de l'adaptateur AA'
<400> 8
   gacgatgagt cctgac 16
<210> 9
   <211> 18
   <212> DNA
   <213> artificial sequence
<220>
   <223> brin A' de l'adaptateur AA'
   <220>
   <221> misc_feature
   <222> (1)..(1)
   <223> phosphate
<400> 9
   cggtcaggac tcatcgtc 18
<210> 10
   <211> 18
   <212> DNA
   <213> artificial sequence
<220>
   <223> brin C de l'adaptateur CC'
   <220>
   <221> misc_feature
   <222> (1)..(1)
   <223> phosphate
<400> 10
   aattggtacg cagtctac 18
<210> 11
   <211> 14
   <212> DNA
   <213> artificial sequence
<220>
   <223> brin C' de l'adaptateur CC'
<400> 11
   gtagactgcg tacc 14
<210> 12
   <211> 18
   <212> DNA
   <213> artificial sequence
<220>
   <223> amorce sens
   <220>
   <221> misc_feature
   <222> (1)..(1)
   <223> Cy3
<400> 12
   gacgatgagt cctgaccg 18
<210> 13
   <211> 18
   <212> DNA
   <213> artificial sequence
<220>
   <223> amorce anti-sens
   <220>
   <221> misc_feature
   <222> (1)..(1)
   <223> biotine
<400> 13
   gtagactgcg taccaatt 18

## Description

L'invention concerne un procédé de préparation de fragments d'ADN par fragmentation sélective d'acides nucléiques et ses applications pour l'analyse des génomes et des transcriptomes.

Les techniques d'analyse des génomes et des transcriptomes d'espèces (animaux, végétaux, microorganismes) différentes ou bien de sous-groupes ou d'individus différents au sein de ces espèces, reposent sur la détection de marqueurs ou d'empreinte(s) génétique(s) par fragmentation de l'ADN (génome ou ADNc) à l'aide d'une ou plusieurs enzymes de restriction puis analyse, par tout moyen approprié des fragments d'ADN ainsi obtenus.

Ces techniques ont des applications dans des domaines extrêmement variés de la biologie comme la cartographie génétique, le génotypage d'espèces, de variétés, d'individus (animaux, végétaux, microorganismes), la détection de polymorphisme(s) (SNP ou *Single Nucleotide Polymorphism*) des gènes lié(s) à des caractères phénotypiques, notamment à des maladies, ainsi que l'établissement de profils d'expression géniques.

Toutefois, du fait notamment de la complexité des génomes, les techniques proposées ne permettent pas une analyse systématique à haut-débit des génomes et des transcriptomes par des techniques automatisées du type hybridation sur des puces à ADN. En effet :
- la technique RFLP (*Restriction Fragment Length Polymorphism*) qui comprend l'analyse par Southern-blotting des fragments générés par les enzymes de restriction possède une faible résolution, dans la mesure où elle ne permet d'analyser qu'un seul ou, au plus, quelques *loci* en une seule réaction. En outre, les fragments obtenus ne peuvent pas être analysés par hybridation sur des puces à ADN du fait du nombre trop important de fragments générés, qui entraîne une saturation de la puce.
- La méthode GSTs (Genomic Signature Tags ; Dunn et al., Genome Research, 2002, 12, 1756-1765), analogue à la méthode SAGE (Serial Analysis of Gene Expression ; Velculescu et al., Science, 1995, 270, 484-487) repose sur l'analyse d'un génome par clonage et séquençage d'un ensemble de courts fragments d'ADN spécifiques (*tags* ou étiquettes de 21 pb) générés par : (i) digestion de l'ADN génomique, successivement avec une première puis une seconde enzyme de restriction de type II, (ii) ligation de l'une des extrémités des fragments de restriction ainsi obtenus avec un adaptateur complémentaire de l'extrémité cohésive générée par la seconde enzyme de restriction de type II, ledit adaptateur contenant la totalité de la séquence du site de reconnaissance d'une enzyme de restriction de type IIS (*Mme* I), et (iii) libération des étiquettes de 21 pb par digestion avec l'enzyme de restriction de type IIS. Cette technique est lourde à mettre en oeuvre puisqu'elle implique le clonage et le séquençage de l'ensemble des fragments obtenus, afin d'identifier le génome à analyser.
- la technique AFLP (*Amplified Fragment Length Polymorphism*) décrite dans la Demande de brevet européenne (EP 0 534 858) au nom de KEYGENE, qui a été adaptée à l'analyse sur puces à ADN (Jaccoud et al., N.A.R., 2001, 29, 4e25), permet de réduire la complexité de l'échantillon de départ à une centaine de fragments par amplification sélective d'une fraction des fragments, par PCR à l'aide d'amorces comprenant en 3' de la séquence du site de restriction, une séquence spécifique de quelques bases (environ 1 à 10). Ainsi une paire d'amorces possédant n bases sélectives permet en théorie d'amplifier uniquement une fraction 1/4²ⁿ des fragments correspondant à ceux qui possèdent une séquence complémentaire de la séquence sélective, soit 1/16^{ème} et 1/256^{ième} des fragments pour respectivement n = 1 et n = 2.
- la technique d'amplification sélective de l'ADN par ligation d'adaptateurs (*Ligation-mediated PCR technique*) décrite notamment dans la Demande EP 0735 144 au nom de RESEARCH DEVELOPMENT CORPORATION OF JAPAN et les articles aux noms de Zheleznaya et al., Biochemistry, 1995, 60, 1037-1043, et Smith et al., PCR Methods and Applications, 1992, 2, 21-27, permet de réduire la complexité de l'échantillon de départ par amplification sélective d'une fraction de fragments de restriction obtenus par clivage avec une enzyme de restriction de type IIS et éventuellement de type IIN, puis ligation avec l'un des adaptateurs complémentaire de l'extrémité cohésive générée par ladite enzyme de type IIS et éventuellement IIN.

Toutefois, malgré la réduction de la complexité de l'échantillon de départ proposée dans les techniques précédentes, l'hybridation des cibles obtenues (produits PCR de plusieurs centaines de paires de bases) sur des supports du type puces à ADN, c'est-à-dire de cibles avec des sondes de 10 à 20 bases est souvent de mauvaise qualité (signaux faibles, faux négatifs et faux positifs) pour les raisons suivantes :
- la présence de structures secondaires dans la cible diminue l'efficacité de l'hybridation de la sonde, du fait de la diminution de l'accessibilité à la cible et de l'impossibilité d'optimiser les conditions d'hybridation en raison de la présence d'un grand nombre de fragments, de structures différentes, à hybrider avec une même sonde, et
- des réactions d'hybridation non-spécifique ou d'hybridation croisée avec des séquences « non-cibles » possédant des similarités avec la séquence cible conduisent à des faux positifs qui réduisent la capacité à détecter de faibles quantités de séquences spécifiques et la capacité de discrimination de ces techniques, du fait de l'augmentation du bruit de fond.
   - le Brevet américain 6,258,539 au nom de THE PERKIN-ELMER CORPORATION préconise d'hybrider des cibles de petites tailles (environ 30 paires de bases) sur des supports du type puces à ADN ; les cibles sont générées à partir d'un fragment de restriction représentatif de l'ADNc à analyser, par : (i) ligation d'une des extrémités du fragment avec un adaptateur contenant le site de reconnaissance d'une enzyme de restriction de type IIS puis clivage de l'extrémité 5' dudit fragment par ladite enzyme de type IIS. Cette technique n'est pas adaptée à l'analyse de populations complexes d'acides nucléiques comme des génomes ou des transcriptomes pour lesquels il est impossible d'obtenir un seul fragment de restriction représentatif de chaque molécule d'intérêt à analyser.

Il ressort de ce qui précède qu'il existe un réel besoin de disposer de méthodes d'analyse des génomes et des transcriptomes, mieux adaptées aux besoins de la pratique, notamment en ce qu'elles sont à la fois fiables, reproductibles, sensibles, spécifiques, rapides et simples à mettre en oeuvre. De telles méthodes, permettant ainsi d'analyser simultanément un grand nombre d'échantillons sur des supports du type puces à ADN, seraient donc parfaitement adaptées à l'analyse systématique des génomes et des transcriptomes pour les applications précitées.

C'est la raison pour laquelle les Inventeurs ont mis au point un procédé de préparation de fragments d'ADN par fragmentation sélective d'acides nucléiques (ADN génomique, ADNc rétro-transcrit à partir d'ARNm) qui permet avantageusement d'obtenir un ou plusieurs ensemble(s) de courts fragments d'ADN (inférieurs à 100 bases ou 100 paires de bases) représentatif(s) de la totalité du génome ou du transcriptome à analyser ; ce procédé permet ainsi d'obtenir une hybridation à la fois rapide, efficace, fiable, reproductible, sensible et spécifique de molécules d'acides nucléiques cibles (ADN, ARN) avec des sondes oligonucléotidiques immobilisées sur des supports miniaturisés du type puce à ADN ; ledit procédé est utile aussi bien pour la préparation d'ADN-cibles aptes à s'hybrider avec des sondes nucléotidiques et en particulier avec des sondes oligonucléotidiques immobilisées sur des supports miniaturisés du type puce à ADN (détection de marqueur(s) ou d'empreinte(s) génétique(s)), que pour la préparation de sondes à ADN, notamment de puces à ADN aptes à s'hybrider avec des acides nucléiques cibles (ADN, ARN), (préparation de marqueur(s) ou d'empreinte(s) génétique(s)).

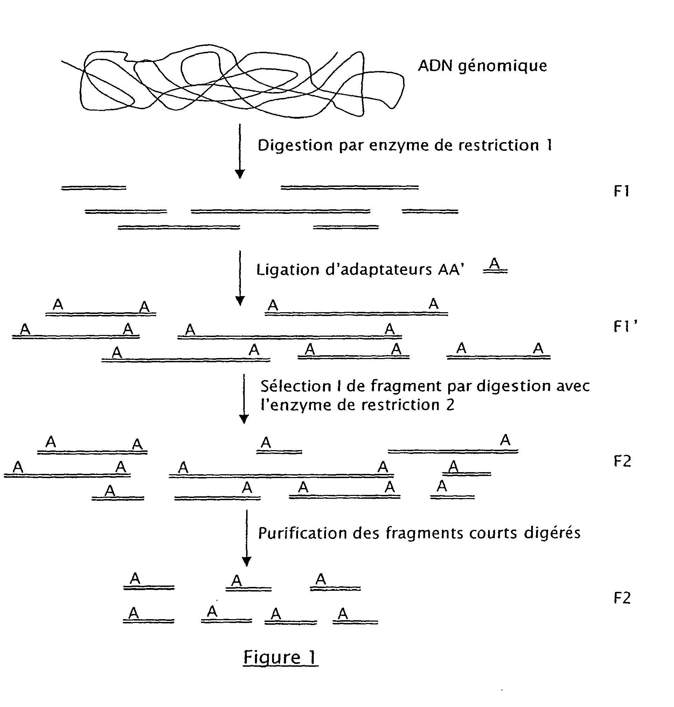

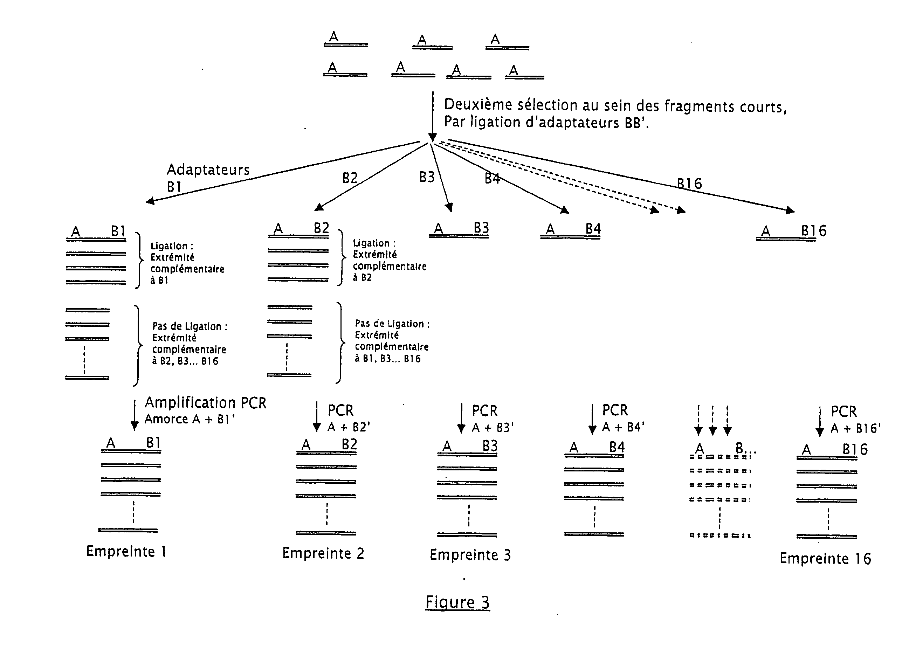

La présente invention a ainsi pour objet un procédé de préparation de fragments d'ADN, à partir d'un échantillon d'acides nucléiques à analyser, lequel procédé est caractérisé en ce qu'il comprend la fragmentation sélective desdits acides nucléiques à l'aide d'au moins les étapes suivantes (figure 1) :
I. Une première sélection de fragments courts comprenant :
   a) la préparation de premiers fragments d'ADN double-brin F1 à l'aide d'au moins une enzyme de restriction E1, apte à fragmenter de manière aléatoire l'échantillon d'acides nucléiques à analyser, en générant lesdits fragments F1 d'ADN aux extrémités franches ou cohésives,
   b) l'obtention de fragments d'ADN F'1 par ligation des extrémités desdits fragments d'ADN F1 obtenus à l'étape a) à au moins un adaptateur AA', de manière à former un motif situé à la jonction de l'extrémité 3' dudit adaptateur et de l'extrémité 5' desdits fragments F1, tel que la séquence dudit motif qui correspond à la fin du site de reconnaissance de l'enzyme de restriction E1, contient les premières paires de bases du site de reconnaissance d'une enzyme de restriction E2 dont le site de coupure est situé en aval dudit site de reconnaissance,
   c) la sélection d'une fraction de fragments courts F2 par coupure, à l'aide de ladite enzyme de restriction E2, de la fraction de fragments F'1 d'ADN obtenus en b) qui ont restauré la totalité du site de reconnaissance de ladite enzyme de restriction E2 par ligation de leurs extrémités audit adaptateur AA',
   d) la purification par tout moyen approprié de ladite fraction de fragments courts F2, et éventuellement,
II. Une deuxième sélection d'un ou plusieurs sous-ensembles de fragments à partir de la fraction de fragments courts F2 obtenue à l'étape d), conformément aux étapes suivantes (figure 3) :
   e) la ligation de l'extrémité libre (non liée à l'adaptateur AA') de fragments courts F2 obtenus en d) à au moins un second adaptateur complémentaire BB' (obtention de fragments F'2), et
   f) l'amplification des fragments courts F'2 liés auxdits adaptateurs (AA' et BB'), à l'aide d'au moins un couple d'amorces appropriées, l'une au moins étant éventuellement marquée à son extrémité 5', de façon à sélectionner au moins un sous-ensemble de fragments courts F'2, à partir de la fraction de fragments courts F2 obtenue en d).

Au sens de la présente invention on entend par :
- fragment d'ADN, un fragment d'ADN double-brin,
- fragment court F2 ou F'2, un fragment d'ADN inférieur à 100 paires de bases,
- fragment long F1 ou F'1, un fragment d'ADN de plusieurs centaines de paires de bases,
- adaptateur, un oligonucléotide double brin d'au moins 6 paires de bases,
- extrémité 5' et extrémité 3', relativement à un fragment d'ADN, un adaptateur ou un site de reconnaissance ou de coupure d'une enzyme de restriction, respectivement l'extrémité 5' et l'extrémité 3' du brin positif dudit fragment d'ADN, dudit adaptateur ou dudit site de reconnaissance ou de coupure d'une enzyme de restriction,
- extrémité libre, relativement à un fragment d'ADN, l'extrémité qui n'est pas liée à un adaptateur,
- extrémité complémentaire d'un adaptateur, l'extrémité dudit adaptateur qui se lie à l'extrémité 3' ou 5' d'un fragment d'ADN ; lorsque ledit adaptateur se lie à l'extrémité 5' dudit fragment d'ADN, il s'agit de l'extrémité 3' dudit adaptateur et inversement,
- fraction de fragments, une fraction de fragments courts F2 préparée à partir des fragments (longs) F1 obtenus à l'étape a) ou une fraction de fragments courts F'2, préparée à partir des fragments courts F2 ; les termes fraction, ensemble ou groupe sont considérés comme équivalents et employés indifféremment dans ce qui suit et il en va de même pour les termes sous-ensemble(s) et sous-groupe(s),
- site de coupure, le site de restriction d'une endonucléase (enzyme de restriction) ; dans ce qui suit le terme site de coupure ou site de restriction sont employés indifféremment.

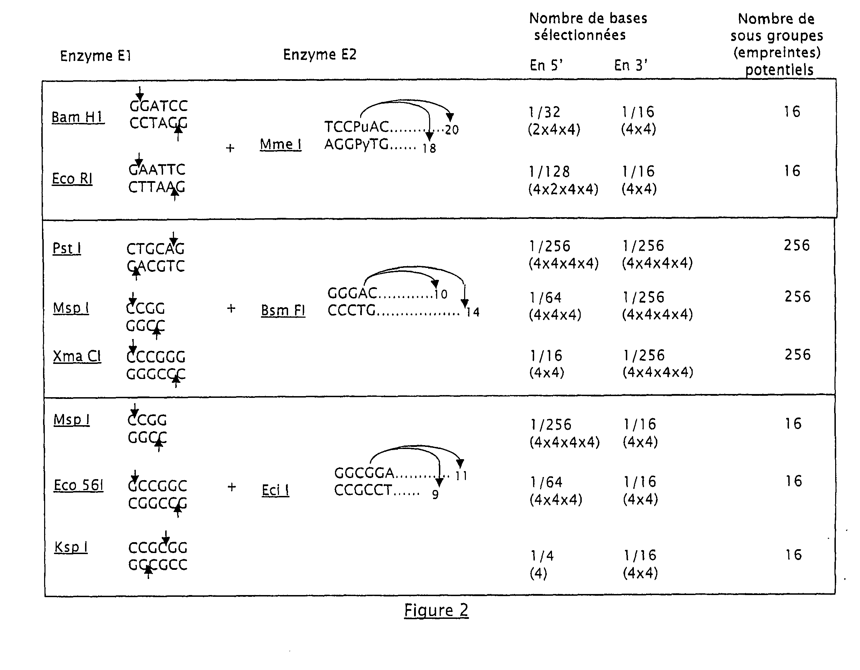

La combinaison des étapes a) à d) du procédé de préparation de fragments d'ADN selon l'invention permet avantageusement à la fois :
- d'obtenir des fragments courts F2 représentatifs de la totalité du génome ou du transcriptome à analyser, c'est-à-dire d'une longueur équivalente à celle des sondes oligonucléotidiques (figure 1), et
- de réduire la complexité de l'échantillon à analyser par une sélection à l'aide des adaptateurs AA' d'une fraction de fragments courts F2 représentatifs du génome ou du transcriptome à analyser (figure 2) ; une telle sélection permet d'éviter les problèmes de saturation du support du type puce à ADN utilisé pour l'hybridation.

La combinaison des étapes a) à f) du procédé de préparation de fragments d'ADN selon l'invention permet avantageusement à la fois (figure 2) :
- d'obtenir des fragments courts F'2 représentatifs de la totalité du génome ou du transcriptome à analyser, c'est-à-dire d'une longueur équivalente à celle des sondes oligonucléotidiques (figures 1 et 3) ;
- de réduire la complexité de l'échantillon à analyser par une première puis une deuxième sélection à l'aide des adaptateurs AA' et BB', d'un ou plusieurs sous-ensembles de fragments courts F'2 représentatifs du génome ou du transcriptome à analyser (figure 2) ; une telle sélection permet d'éviter les problèmes de saturation du support du type puce à ADN utilisé pour l'hybridation, et
- de détecter à partir de l'ensemble de fragments courts F2 un nombre maximum d'empreintes ou de marqueurs génétiques différents représentatifs du génome ou du transcriptome à analyser, par une deuxième sélection de sous-ensembles de cet ensemble de fragments courts F2 obtenus à l'étape d), à l'aide d'adaptateurs différents (B₁B₁', B₂B₂'..), (figures 2 et 3).

L'utilisation de tels fragments courts F2 ou F'2 comme cibles ou sondes dans des techniques d'hybridation sur puces à ADN, présente les avantages suivants par rapport aux techniques d'analyse de génomes ou de transcriptomes de l'art antérieur :

### • fiabilité et reproductibilité

La fraction de fragments courts F2 qui est sélectionnée uniquement par ligation d'adaptateurs et coupure par des enzymes de restriction est représentative de la totalité du génome ou du transcriptome à analyser. Ces fragments courts d'une part sont plus faciles à amplifier et d'autre part, permettent de travailler sur de l'ADN partiellement dégradé. En outre, la réduction de la complexité de l'échantillon à analyser par une première sélection, à l'aide de l'adaptateur AA', d'une fraction de fragments courts F2 représentatifs du génome ou du transcriptome à analyser, qui permet d'éviter les problèmes de saturation du support du type puce à ADN utilisé pour l'hybridation, contribue également à augmenter la fiabilité et la reproductibilité de l'analyse des génomes ou des transcriptomes.

### • sensibilité et spécificité

La sensibilité et la spécificité de l'hybridation sont augmentées du fait de :
- la réduction de la taille des fragments à hybrider (cibles ou sondes de moins de 100 bases ou paires de bases au lieu de plusieurs centaines de bases ou de paires de bases dans les techniques de l'art antérieur) ; cette réduction diminue les réactions d'hybridation croisée et les faux positifs par élimination des « séquences non-cibles », et augmente le signal d'hybridation par diminution des structures secondaires de l'ADN,
- l'harmonisation des conditions d'hybridation (température) pour des fragments de taille homogène,
- la pureté de l'ADN (élimination de l'enzyme, des tampons et des longs fragments d'ADN restants.)

### • simplicité

La fragmentation des acides nucléiques (cible ou sonde) comprend des étapes simples à mettre en oeuvre (digestion enzymatique, ligation). En outre, l'optimisation de la longueur, de la structure et de la composition de l'ADN (cible ou sonde) permet d'obtenir une hybridation de bonne qualité (pas de faux positifs, peu de bruit de fond..) et donc de minimiser le nombre de contrôles nécessaires et par conséquent de réduire la complexité de la puce.

### • rapidité

La durée d'hybridation est réduite de façon importante et est inférieure à 1 h (environ 15 à 20 min), au lieu de 12 h à 18 h dans les techniques de l'art antérieur.

### • coût peu élevé

La réduction de la complexité de la puce permet de réduire le coût de cette dernière.

En raison de ces différents avantages, le procédé de préparation de fragments d'ADN par fragmentation sélective d'acides nucléiques selon l'invention est particulièrement bien adapté à :
- l'analyse rapide d'un grand nombre d'échantillons d'acides nucléiques-cibles (ADN génomique ou ADNc obtenu par rétro-transcription d'ARNm) sur des puces à ADN, et
- la préparation de sondes de taille réduite et contrôlée à partir d'ARN
ou d'ADN génomique, notamment pour la fabrication de puces à ADN sur lesquelles sont immobilisées lesdites sondes représentant des marqueurs génétiques de génomes ou de transcriptomes.

Conformément au procédé de l'invention, les fragments F1 d'ADN double-brin de l'étape a) sont obtenus par les techniques classiques connues en elles mêmes. Par exemple, l'ADN génomique extrait de l'échantillon à analyser est fragmenté de façon aléatoire à l'aide d'une ou plusieurs enzymes de restriction E1 générant des fragments aux extrémités franches ou cohésives, sélectionnée(s) en fonction de leur fréquence de coupure de l'ADN à analyser, de façon à obtenir des fragments inférieurs à 1000 pb, de l'ordre de 200 à 400 pb. L'ARN (ARNm, ARN génomique d'un microorganisme...) est extrait de l'échantillon à analyser, converti en ADNc double brin par rétro-transcription puis fragmenté de façon analogue à l'ADN génomique. Parmi les endonucléases de restriction E1 utilisables pour couper l'ADN de mammifères, on peut citer de façon non-limitative : *EcoR I, BamH I, Pst I, Msp I, XmaC I, Eco 561, Ksp I, Dra I, Ssp I, Sac I, BbνC I, Hind III, Sph I, Xba I et Apa I.*

Conformément à l'invention l'enzyme E1 génère soit des extrémités franches, soit des extrémités cohésives ; elle génère de préférence des extrémités cohésives qui ont l'avantage de permettre la ligation avec un seul adaptateur.

Conformément au procédé de l'invention, l'adaptateur tel que défini à l'étape b) est un oligonucléotide d'au moins 6 pb, formé de deux brins complémentaires (A et A') ; ledit adaptateur, en b), est lié aux extrémités dudit fragment d'ADN F1 par tout moyen approprié, connu en lui-même, notamment à l'aide d'une ligase à ADN, telle que la T4 ligase.

Conformément au procédé de l'invention, les étapes a) et b) sont réalisées successivement ou simultanément.

Conformément au procédé de l'invention, l'extrémité 3' du site de coupure de l'enzyme de restriction E1 et l'extrémité 5' du site de reconnaissance de l'enzyme de restriction E2 se chevauchent sur au moins une paire de bases (figure 2), ce qui permet de sélectionner une fraction de fragments courts F2 par coupure avec l'enzyme de restriction E2 ; ces fragments courts F2 sont issus de la fraction de fragments longs F'1 obtenus à l'étape b), qui comprend la totalité du site de reconnaissance de ladite enzyme de restriction E2 (N paires de bases). Parmi les enzymes de restriction E2, on peut citer de façon non-limitative : *Bpm I, Bsg I* et *BpuE I* qui coupent 16 nucléotides en aval de leur site de reconnaissance, et *Eci I*, *BsmF I*, *Fok I*, *Mme I et Mbo II* qui coupent respectivement 11, 10, 9, 20 et 8 nucléotides en aval de leur site de reconnaissance. Conformément au procédé de l'invention, le chevauchement desdits sites peut-être parfait (absence de misappariement) ou il peut comprendre au moins un misappariement (voir par exemple la paire de bases située en deuxième position du site *Ksp I* (enzyme de restriction E1) qui n'est pas complémentaire de la paire de base en première position du site *Eci I* (enzyme de restriction E2), figure 2) ; dans ce cas, la séquence du site de reconnaissance de l'enzyme de restriction E2 est restaurée par ligation avec un adaptateur dont l'extrémité est complémentaire dudit site de reconnaissance de l'enzyme de restriction E2 (adaptateur comprenant la séquence « GGC » à l'extrémité 3' du brin A dans l'exemple précité).

Le nombre 1 à N-1 de paires de bases du site de reconnaissance de l'enzyme de restriction E2 situées dans la région de la jonction de l'extrémité 3' complémentaire dudit adaptateur AA' et de l'extrémité 5' desdits fragments d'ADN F1 et la longueur dudit site de reconnaissance de l'enzyme de restriction E2, déterminent la fraction de fragments courts qui peut être sélectionnée à partir de l'ensemble des fragments (longs) F1 générés à l'étape a) ; pour un site de reconnaissance E2 de N pb et un chevauchement de n pb, entre E1 et E2, la fraction de fragments sélectionnés correspond à 1/4^{(N-n)}, cette valeur étant augmentée d'un multiple de 2 pour toute paire de bases puriques ou pyrimidiques reconnue indifféremment par ladite enzyme de restriction E2 (enzyme *Mme I,* figure 2). Ainsi, plus le chevauchement est important, plus le nombre de fragments contenus dans la fraction est grand (facteur de sélection
ou de réduction de la complexité de l'échantillon faible), et inversement (facteur de sélection ou de réduction de la complexité de l'échantillon élevé), (figure 2).

Conformément au procédé de l'invention, la coupure à l'extrémité 5' des fragments longs F'1 à l'étape c), permet d'obtenir de courts fragments d'ADN F2 représentatifs du génome ou du transcriptome à analyser, susceptibles de contenir un marqueur génétique apte à être détecté par hybridation avec une sonde nucléotidique spécifique, en particulier un oligonucléotide complémentaire dudit marqueur génétique. Alternativement, lesdits fragments sont immobilisés sur un support solide du type puce à ADN et utilisés comme empreinte ou marqueur génétique pour analyser des génomes ou des transcriptomes.

Conformément au procédé de l'invention, la purification des fragments courts F2 -éventuellement simple-brin et/ou liés à un marqueur approprié (biotine, digoxygénine, fluorescéine)- (étape d), est réalisée par tout moyen approprié connu en lui-même, par exemple : la chromatographie d'exclusion, la filtration, la précipitation à l'aide de mélanges d'éthanol et d'acétate d'ammonium ou de sodium, la liaison à un support fonctionnalisé (billes magnétiques, billes de polymère non-magnétique ou surface d'or, couplées notamment à la streptavidine ou à un anticorps anti-digoxygénine, anti-fluorescéine ).

Selon un mode de mise en oeuvre avantageux du procédé selon l'invention, l'étape a) est réalisée avec deux enzymes de restriction E1 différentes, E1_{A} et E1_{C}, telles que :
- l'une au moins génère des extrémités cohésives, distinctes de celles éventuellement générées par l'autre enzyme de restriction, et
- l'extrémité 3' du site de restriction de E1_{1A} est celle du motif tel que défini à l'étape b).

Selon une disposition avantageuse de ce mode de mise en oeuvre, l'une des enzymes coupe fréquemment et l'autre rarement.

De préférence, l'enzyme qui coupe fréquemment est l'enzyme E1_{A}, laquelle enzyme E1_{A} génère au moins une extrémité d'un fragment F1, qui se lie à l'adaptateur AA' à l'étape b). L'enzyme E1_{A} est liée à l'enzyme E2 dans la mesure où, l'extrémité 3' du site de restriction de E1_{A} correspond aux N-x premières paires de bases du site de reconnaissance de E2. L'enzyme E1_{C} génère au moins une extrémité d'un fragment F1-identique ou différent de celui généré par l'enzyme E1_{A}, laquelle extrémité se lie, à l'étape b), à un second adaptateur CC' qui est différent de l'adaptateur AA'. De manière préférée, l'extrémité 3' du site de restriction de E1_{C} est différente de celle des N-x premières paires de bases du site de reconnaissance de l'enzyme E2, telle que définie à l'étape b), de manière à ne pas reconstituer la séquence des N-x premières bases ou paires de bases du site de reconnaissance de l'enzyme de restriction E2, par ligation dudit adaptateur CC' à l'une au moins des extrémités desdits fragments d'ADN obtenus en a).

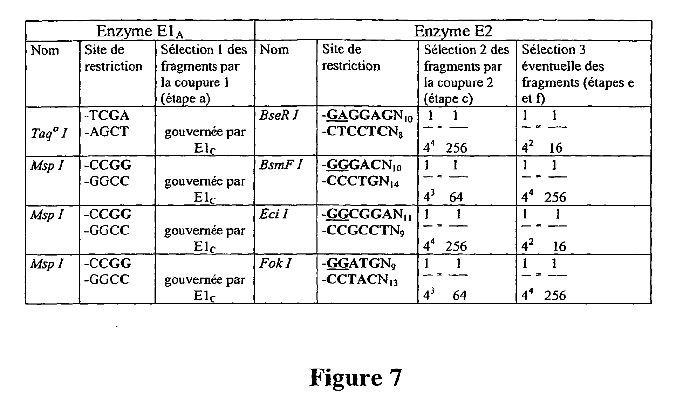

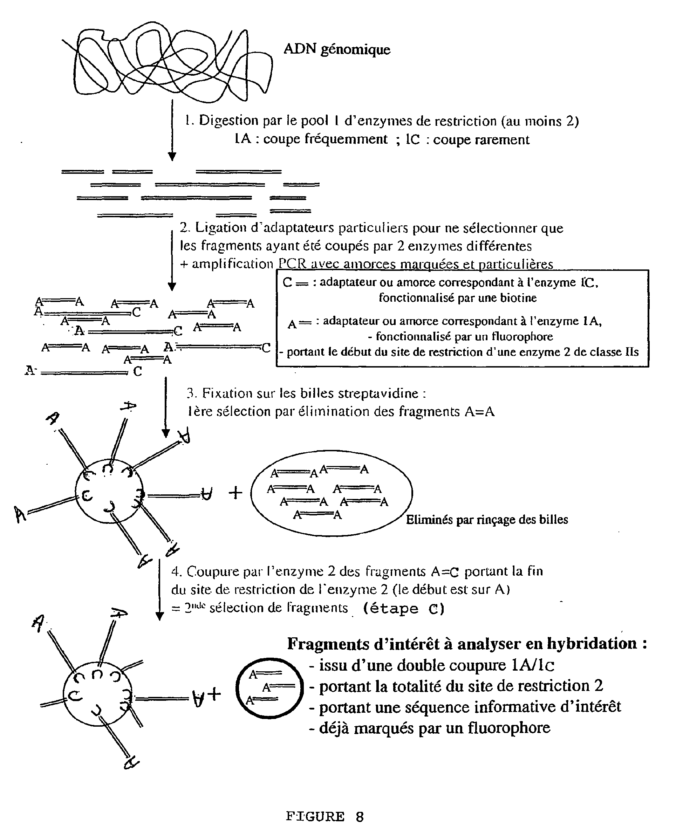

L'utilisation d'un tel couple d'enzymes, permet de réduire encore d'avantage la complexité de l'échantillon à analyser par une sélection supplémentaire d'un ensemble de fragments A=C, notamment par liaison à un support fonctionnalisé par un ligand du marqueur lié à l'extrémité 5' de l'adaptateur CC' (figures 7 et 8).

A titre d'exemple non-limitatif d'enzymes qui coupent l'ADN fréquemment, on peut citer celles dont le site de restriction possède 4 paires de bases comme *Msp I* et *Taq^{α} I*.

A titre d'exemple non-limitatif d'enzymes qui coupent l'ADN rarement, on peut citer celles dont le site de restriction possède 5 ou 6 paire de bases, comme *Pst I* et *EcoR I*. Selon un mode de mise en oeuvre avantageux du procédé selon l'invention, il comprend une étape additionnelle de purification des fragments inférieurs à 1000 pb, préalablement à l'étape b) de ligation. Ladite purification est effectuée par tout moyen approprié connu en lui-même, notamment par séparation des produits de digestion obtenus en a) par électrophorèse en gel d'agarose, visualisation des bandes correspondant aux différents fragments obtenus, prélèvement de(s) bande(s) du gel correspondant aux fragments inférieurs à 1000 pb et extraction desdits fragments d'ADN double brin selon les techniques classiques.

Selon un autre mode de mise en oeuvre avantageux du procédé selon l'invention, l'adaptateur AA' tel que défini à l'étape b) comprend à l'extrémité 3' du brin A et/ou 5' du brin A', une zone 1 d'environ 1 à 8 bases ou paires de bases, identique ou complémentaire - en partie ou en totalité- au site de coupure de l'enzyme E1 ou E1_{A}, choisie de manière à reconstituer la séquence des N-x premières bases ou paires de bases du site de reconnaissance de l'enzyme de restriction E2, par ligation dudit adaptateur AA' à l'une au moins des extrémités desdits fragments d'ADN obtenus en a). Ladite zone 1 peut éventuellement inclure un ou plusieurs misappariements avec la séquence dudit site de coupure de l'enzyme E1.

Selon encore un autre mode de mise en oeuvre avantageux du procédé selon l'invention, l'adaptateur CC' tel que défini ci-dessus comprend à l'extrémité 3' du brin C et/ou 5' du brin C', une zone 1 d'environ 1 à 8 bases ou paires de bases, complémentaire - en partie ou en totalité- au site de coupure de l'enzyme E1_{C} ; ladite zone 1 peut éventuellement inclure un ou plusieurs misappariements avec la séquence dudit site de coupure de l'enzyme E1_{C}. Ladite zone 1 qui est différente de la zone 1 de l'adaptateur AA' est choisie de manière à : (i) lier uniquement l'extrémité générée par l'enzyme E1_{C} mais pas celle générée par l'enzyme E1_{A} et (ii) ne pas reconstituer la séquence des N-x premières bases ou paires de bases du site de reconnaissance de l'enzyme de restriction E2, par ligation dudit adaptateur CC' à l'une au moins des extrémités desdits fragments d'ADN obtenus en a).

Selon encore un autre mode de mise en oeuvre avantageux du procédé selon l'invention, l'adaptateur AA' tel que défini à l'étape b) ou l'adaptateur CC' tel que défini ci-dessus, comprennent en amont de la zone 1, une zone 2 d'au moins 6 paires de bases qui permet d'améliorer l'hybridation par allongement de l'adaptateur. La séquence de cette zone 2 est sélectionnée par tout moyen approprié connu en lui même, notamment à l'aide de logiciels de prédiction de séquences appropriés permettant d'optimiser la longueur, la structure et la composition des oligonucléotides (pourcentage de GC, absence de structures secondaires et/ou d'auto-appariement...) ; de préférence, ledit adaptateur comprend au moins une base située entre la zone 1 et la zone 2, différente de celle qui dans le site de coupure de l'enzyme de restriction E1, est immédiatement adjacente à la séquence complémentaire précédente ; cette base permet de ne pas reconstituer ledit site de restriction après la ligation de l'adaptateur à l'étape b) et donc d'éviter le clivage de l'adaptateur lié à l'extrémité dudit fragment d'ADN double-brin.

Selon encore un autre mode de mise en oeuvre avantageux du procédé selon l'invention, l'adaptateur AA' tel que défini à l'étape b) et/ou l'adaptateur CC' tel que défini ci-dessus comprennent un résidu de phosphate lié de façon covalente à l'extrémité 5' du brin A' et/ou C ; ce résidu de phosphate permet à une enzyme comme la T4 ADN ligase de lier ledit adaptateur aux extrémités 3'-OH du fragment d'ADN double brin (F1), par l'intermédiaire d'une liaison phosphodiester.

Selon encore un autre mode de mise en oeuvre avantageux du procédé selon l'invention, l'adaptateur AA' tel que défini à l'étape b) et/ou l'adaptateur CC' tel que défini ci-dessus sont liés à l'extrémité 5' du brin A et/ou C' à des marqueurs différents

Selon une disposition avantageuse de ce mode de mise en oeuvre, l'extrémité 5' du brin C' de l'adaptateur CC' est liée à un marqueur qui peut se fixer sur un support solide fonctionnalisé.

Les supports solides fonctionnalisés permettant de fixer les acides nucléiques sont connus de l'Homme du métier. A titre d'exemple non-limitatif, on peut citer notamment les billes magnétiques fonctionnalisées par la streptavidine (liaison à une molécule d'acide nucléique marquée par la biotine), un anticorps anti-fluorescéine ou anti-digoxygénine (liaison à une molécule d'acide nucléique marquée par la fluorescéine ou la digoxygénine), ou bien encore d'autres support fonctionnalisés tels que des billes de polymère non-magnétiques ou une surface d'or, fonctionnalisées.

Selon une autre disposition avantageuse de ce mode de mise en oeuvre, l'adaptateur AA' est lié à un marqueur permettant la détection d'hybrides d'acides nucléiques (ADN-ADN ou ADN-ARN), par exemple un fluorophore.

Selon encore un autre mode de mise en oeuvre avantageux du procédé selon l'invention, lorsque ledit procédé comprend une seule sélection de fragments courts selon les étapes a) à d) telles que définies ci-dessus, il comprend au moins une étape additionnelle b'), c') et/ou d'), respectivement entre les étapes b) et c) ou c) et d), ou bien après l'étape d), d'amplification des fragments F'1 ou F2 à l'aide d'un couple d'amorces appropriées, de préférence un couple d'amorces marquées à l'aide d'un marqueur tel que défini ci-dessus.

De préférence, les fragments F'1 sont amplifiés à l'aide d'un couple d'amorces AA' ou AC' dans lequel la séquence des amorces A, A' et C' est celle de l'un des brins des adaptateurs AA' et CC' tels que définis ci-dessus, l'amorce A et/ou l'amorce C' étant éventuellement liées en 5', respectivement par un marqueur permettant la détection d'hybrides d'acides nucléiques (ADN-ADN ou ADN-ARN) et un marqueur qui peut se fixer sur un support solide fonctionnalisé, tels que définis ci-dessus. De préférence, les fragments courts F2 sont liés en 3' avec un mélange d'adaptateurs complémentaires de l'ensemble des extrémités 3' des dits fragments F2 susceptibles d'être générés par ladite enzyme de restriction E2, puis lesdits fragments courts F2 sont amplifiés à l'aide d'une amorce (sens) A telle que définie ci-dessus, de préférence liée en 5' à un marqueur permettant la détection d'hybrides d'acides nucléiques (ADN-ADN ou ADN-ARN), et d'un mélange d'amorces anti-sens correspondant au mélange des séquences de l'un des brins du mélange d'adaptateur précédent.

Selon encore un autre mode de mise en oeuvre avantageux du procédé selon l'invention, lorsque les étapes a) et b) sont réalisées, respectivement avec deux enzymes de restriction différentes E1_{A} et E1_{C} et deux adaptateurs différents AA' et CC' tels que l'adaptateur AA' ou CC' est lié à un marqueur qui peut se fixer sur un support solide fonctionnalisé, les fragments F'1 obtenus à l'étape b) ou b') sont mis en contact avec ledit support fonctionnalisé préalablement à l'étape c) de coupure, et la fraction de fragments courts F2 de l'étape d) correspond, à la fraction de fragments qui est, soit retenue sur ledit support (adaptateur AA' lié au marqueur qui se fixe sur le support), soit libre (adaptateur CC' lié au marqueur qui se fixe sur le support).

Ladite fraction libre est récupérée par tout moyen connu de l'Homme du métier, notamment par centrifugation ou aimantation du support fonctionnalisé (billes).

Ladite fraction retenue sur le support peut éventuellement être récupérée par dénaturation de l'ADN double-brin, notamment par la soude ou bien par amplification à l'aide d'un couple d'amorces appropriées, notamment avec une amorce A sens et un mélange d'amorces anti-sens tel que défini ci-dessus.

Conformément au procédé de l'invention, les dits fragments courts F2 obtenus à l'étape d) comprennent une extrémité constituée par l'adaptateur AA' et l'autre extrémité (extrémité libre) qui est de préférence cohésive, comprend une séquence aléatoire de quelques bases (inférieure à 10) générée par clivage avec l'enzyme de restriction E2 (figure 2) ; en conséquence, il est possible de sélectionner un ou plusieurs sous-ensembles de fragments courts F'2 par ligation avec un adaptateur (BB') ou plusieurs adaptateurs différents (B₁B₁', B₂B₂'..), chacun comprenant à l'extrémité 5' du brin B ou à l'extrémité 3' du brin B', une séquence cohésive spécifique de 1 à 10 bases, complémentaire de l'extrémité 3' d'un sous-ensemble de fragments courts F'2 (figure 3). Le ou lesdits sous-ensembles de fragments F'2 sont amplifiés, de façon indépendante ou simultanée, par PCR à l'aide d'un couple d'amorces dont la séquence est complémentaire de celle des brins A et B' des adaptateurs tels que définis ci-dessus.

Conformément au procédé de l'invention, l'adaptateur BB' tel que défini à l'étape e) est un oligonucléotide d'au moins 6 pb, formé de deux brins complémentaires (B et B'), sélectionné par tout moyen approprié connu en lui même, notamment à l'aide de logiciels de prédiction de séquences appropriés permettant d'optimiser la longueur, la structure et la composition des oligonucléotides (pourcentage de GC, absence de structures secondaires et/ou d'auto-appariement...).

Conformément au procédé de l'invention ledit adaptateur en e) est lié aux extrémités desdits fragments courts F2 par tout moyen approprié, connu en lui-même, notamment à l'aide d'une ligase à ADN, telle que la T4 ligase.

Conformément au procédé de l'invention, l'amplification de l'étape f) est réalisée notamment par PCR à l'aide d'un couple d'amorces dont les séquences sens et anti-sens sont respectivement, celles du brin A et du brin B' des adaptateurs tels que définis ci-dessus.

Selon une disposition avantageuse de ce mode de mise en oeuvre, l'étape e) comprend la ligation -de façon simultanée ou indépendante, de préférence indépendante- d'une extrémité des fragments courts F2 obtenus en d) à plusieurs adaptateurs différents (B₁B₁, B₂B₂'..), chacun comprenant -à l'extrémité 5' du brin B ou à l'extrémité 3' du brin B'- une séquence spécifique de 1 à 10 bases, complémentaire de l'extrémité 3' libre dudit fragment court F2. Une telle disposition permet avantageusement d'obtenir des sous-groupes de fragments F'2, chacun correspondant à une empreinte ou à un marqueur génétique différent ; ainsi des adaptateurs possédant des séquences spécifiques de n bases permettent d'obtenir 4ⁿ sous-groupes d'empreintes génétiques différentes (figure 2).

Selon une autre disposition avantageuse de ce mode de mise en oeuvre, ledit adaptateur BB' (étape e) comprend un résidu de phosphate lié de façon covalente à l'extrémité 5' du brin B ; ce résidu de phosphate permet à une enzyme comme la T4 ADN ligase de lier ledit adaptateur à l'extrémité 3'-OH du fragment court F2, par l'intermédiaire d'une liaison phosphodiester.

Selon encore une autre disposition avantageuse de ce mode de mise en oeuvre, l'une des amorces (étape f) est liée à son extrémité 5' à un marqueur approprié permettant la détection d'hybrides d'acides nucléiques (ADN-ADN ou ADN-ARN), par exemple un fluorophore.

Selon encore une autre disposition avantageuse des modes de mises en oeuvre précédents, ils comprennent une étape additionnelle d") ou g) d'obtention, par tout moyen approprié, de fragments simple-brin à partir des fragments courts F2 obtenus à l'étape d) ou d') ou bien des fragments courts F'2 obtenus à l'étape f). De préférence, l'un des brins du fragment court obtenu à l'étape d), d') ou f) est protégé à son extrémité 5' par un marqueur approprié ; un tel marqueur permet notamment d'éliminer le brin complémentaire par l'action de la phosphatase puis de la 5' exonucléase.

Selon encore une autre disposition avantageuse des modes de mises en oeuvre précédents, ils comprennent une étape additionnelle de purification, par tout moyen approprié, des produits d'amplification obtenus à l'étape b'), c'), d') ou f) ou des fragments simple brin obtenus aux étapes d") ou g).

La présente invention a également pour objet un ensemble de fragments courts d'ADN, représentant un marqueur génétique susceptible d'être obtenu par le procédé tel que défini ci-dessus, caractérisé en ce qu'il présente une séquence inférieure à 100 bases ou paires de bases comprenant au moins une séquence spécifique constituée par un fragment d'ADN génomique ou d'ADNc bordé respectivement par le site de reconnaissance et le site de clivage d'une enzyme de restriction E2 dont le site de clivage est situé en aval dudit site de reconnaissance, telle que les premières paires de base du site de reconnaissance de ladite enzyme de restriction E2 correspondent à la fin du site de reconnaissance d'une enzyme de restriction E1 telle que définie ci-dessus et les dernières paires de bases du site de reconnaissance de ladite enzyme E2 correspondent à l'extrémité 5' de ladite séquence spécifique, ledit marqueur incluant à chaque extrémité au moins 6 bases ou 6 paires de bases de séquence non-spécifique.

Selon un mode de réalisation avantageux dudit ensemble de fragments courts d'ADN, il s'agit d'un ensemble de fragments simple-brin.

Selon un autre mode de réalisation avantageux dudit ensemble de fragments courts d'ADN, l'une des extrémités 5' desdits fragments est liée à un marqueur approprié permettant la détection d'hybrides d'acides nucléiques (ADN-ADN ou ARN-ADN), par exemple un fluorophore.

La présente invention a également pour objet un support approprié notamment un support miniaturisé du type puce à ADN comprenant ledit ensemble de fragments courts d'ADN. Les supports sur lesquels on peut immobiliser des acides nucléiques sont connus en eux-mêmes ; à titre d'exemple non-limitatif on peut citer ceux qui sont réalisés dans les matériaux suivants : plastique, nylon, verre, gel (agarose, acrylamide...) et silicium.

Outre les fragments d'ADN tels que définis ci-dessus, l'invention a également pour objet les mélanges de fragments d'ADN correspondant aux sous-ensembles de fragments courts F'2 obtenus à l'étape f) ou g) ; lesdits fragments ou leurs mélanges tels que définis ci-dessus sont utiles comme marqueurs génétiques pour l'analyse de génomes et de transcriptomes, notamment pour la détection d'une espèce, d'une variété ou d'un individu (animal, végétal, microorganisme), la détection de polymorphisme des gènes, et pour l'établissement de profils d'expression géniques.

En conséquence, la présente invention a également pour objet l'utilisation d'un ensemble de fragments courts d'ADN tel que défini ci-dessus ou bien de mélanges de fragments d'ADN correspondant aux sous-ensembles de fragments courts F'2 obtenus à l'étape f) ou g) du procédé tel que défini ci-dessus, comme marqueurs génétiques.

La présente invention a également pour objet une méthode d'hybridation d'acides nucléiques, caractérisée en ce qu'elle met en oeuvre un ensemble de fragments courts d'ADN tel que défini ci-dessus.

La présente invention a également pour objet un kit pour la mise en oeuvre d'une méthode d'hybridation, caractérisé en ce qu'il comprend au moins un ensemble de fragments d'ADN (cible ou sonde) tel que défini ci-dessus ; de préférence, lorsque ledit ensemble de fragments est une cible, ledit kit comprend également un ensemble de molécules d'acide nucléique complémentaires desdits fragments d'ADN, notamment une sonde oligonucléotidique.

La présente invention a également pour objet l'utilisation d'au moins un adaptateur AA' tel que défini ci-dessus, en combinaison avec une enzyme E2 telle que définie ci-dessus, pour la préparation de fragments d'ADN tels que définis ci-dessus.

La présente invention a également pour objet un kit pour la mise en oeuvre du procédé tel que défini ci-dessus, caractérisé en ce qu'il comprend au moins un adaptateur AA' et une enzyme E2 tels que définis ci-dessus ; de préférence, ledit kit comprend également au moins un adaptateur BB' et un couple d'amorces tels que définis ci-dessus.

Outre les dispositions qui précèdent, l'invention comprend encore d'autres dispositions qui ressortiront de la description qui va suivre, qui se réfère à des exemples de mise en oeuvre du procédé selon l'invention et de son utilisation pour l'analyse de génomes par hybridation avec des sondes oligonucléotidiques immobilisées sur un support miniaturisé du type puce à ADN, ainsi qu'aux dessins annexés dans lesquels :
- la figure 1 illustre les étapes a) à d) du procédé selon l'invention ; pour simplifier la figure, seul le brin A de l'adaptateur AA' est annoté,
- la figure 2 illustre à l'aide d'exemples de sites de coupure de l'enzyme de restriction E1 et de sites de reconnaissance de l'enzyme de restriction E2, la fraction de fragments courts F2 qui est sélectionnée à l'étape d) et le nombre de sous-groupes potentiels d'empreintes obtenus à l'étape f), déduits à partir du nombre de bases sélectionnées, respectivement aux extrémités 5' (étape b) et 3' (étape e) desdits fragments courts d'ADN.
- la figure 3 illustre les étapes e) et f) du procédé selon l'invention ; pour simplifier la figure, seuls les brins A et B des adaptateurs AA' et BB' sont annotés,

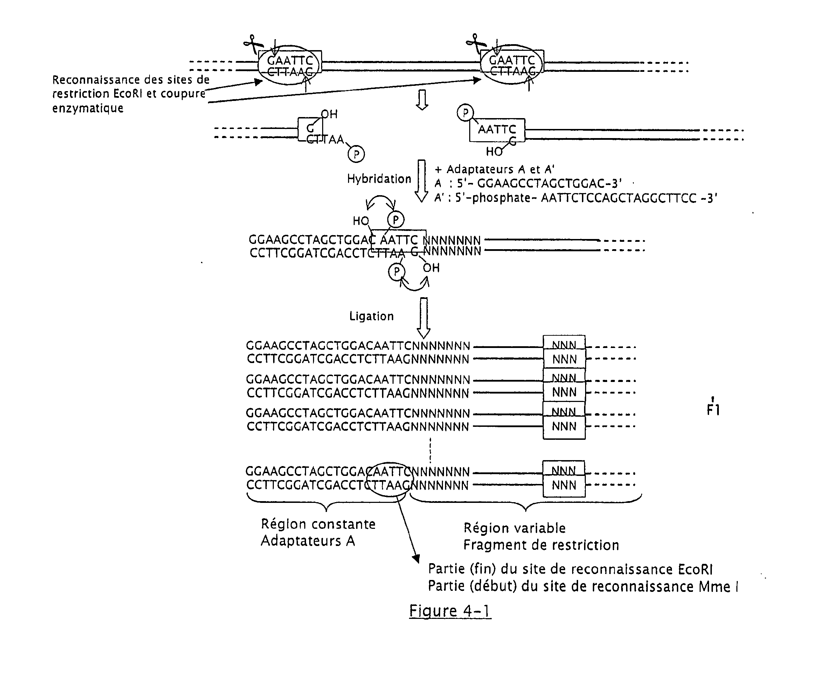

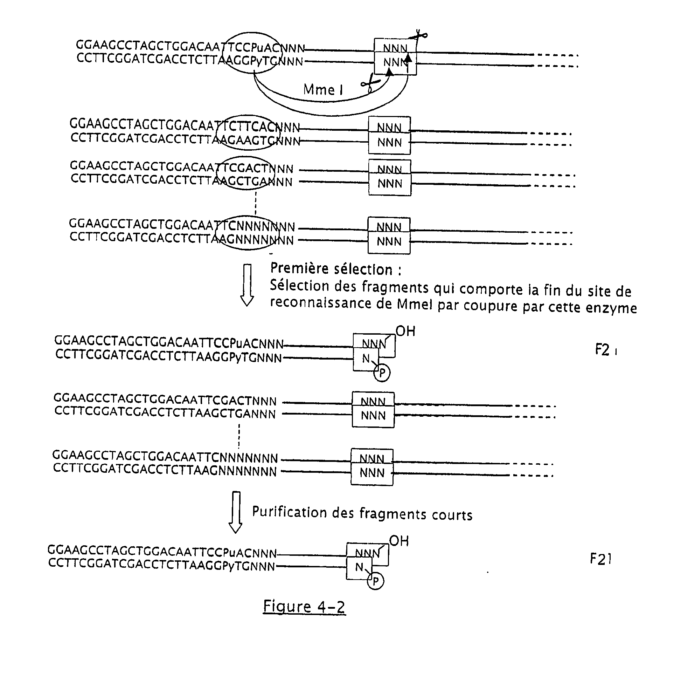

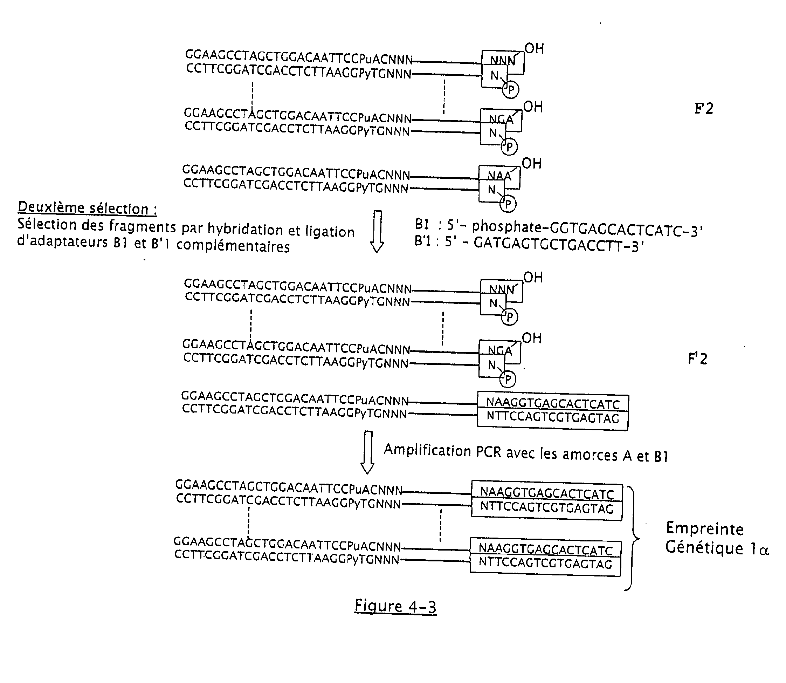

- les figures 4-1 à 4-3 illustrent un premier exemple des étape a) à f) du procédé selon l'invention : figure 4-1 : étapes a et b, figure 4-2 : étapes c et d, figure 4-3 : étapes e et f ;
- étape a) : les fragments d'ADN-double-brin sont générés par coupure avec *EcoR I* qui reconnaît le site GAATTC,
- étape b) : l'adaptateur AA' (16/20 pb), comprend respectivement de 5' en 3': 15 paires de bases (zone 2 : GGAAGCCTAGCTGGA (SEQ ID NO:1) sur le brin A) et 1 pb de base non complémentaire du site *EcoR I* (C sur le brin A), ainsi que 4 bases complémentaires du site *EcoR I* (zone 1) incluant l'extrémité 5' du site *Mme I* (A) et un résidu de phosphate, à l'extrémité 5'du brin A' (5'phosphate-AATT). L'ADN ligase permet la liaison de l'adaptateur aux extrémités cohésives des fragments *EcoR I* par l'intermédiaire de liaisons phosphodiester, et
- étapes c) et d) : les fragments liés à l'adaptateur sont coupés à l'aide de l'enzyme *Mme I* (enzyme E2) de façon à générer des fragments courts (45/43 pb) à partir des fragments qui ont restauré le site *Mme I* (TCCPuAC) par ligation de l'adaptateur AA' avec un fragment dont l'extrémité 5' correspond à la séquence CPuAC ; la sélection de 4 paires de bases spécifiques (CPuAC) permet de diminuer le nombre de fragments d'un facteur 128 (4x2x4x4), par rapport à l'échantillon de départ.
- étape d) : les fragments courts obtenus à l'étape c) sont purifiés,
- étape e) : les fragments courts purifiés à l'étape d) sont liés à l'une de leur extrémité à un adaptateur B₁B₁' (14/16 pb) comprenant 2 bases complémentaires de l'extrémité dudit fragment (TT) à l'extrémité 3' du brin B'₁, et un groupement phosphate à l'extrémité 5' du brin B₁ ; la sélection de 2 bases spécifiques (AA) permet de diminuer le nombre de fragments d'un facteur 2048 (4x2x4x4x16) par rapport à l'échantillon de départ et d'obtenir des fragments courts de 59 paires de bases comprenant 28 paires de bases spécifiques de l'ADN à analyser, lesquels fragments correspondent à 16 sous-groupes potentiels d'empreintes génétiques,
- étape f) : les fragments sélectionnés à l'étape e) sont amplifiés à l'aide d'amorces sens et anti-sens correspondant aux séquences complémentaires de respectivement les brins A et B'₁ des adaptateurs AA' et B₁B'₁.

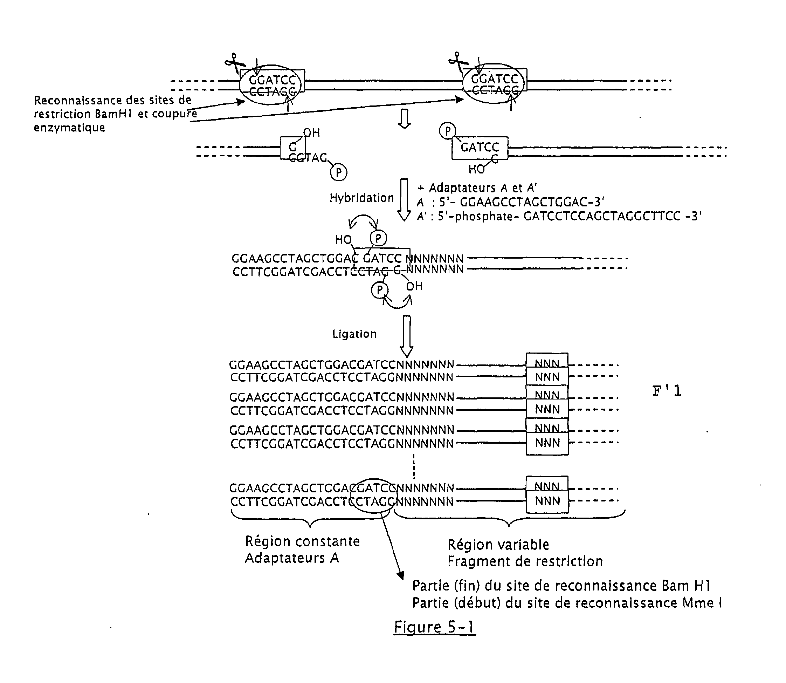

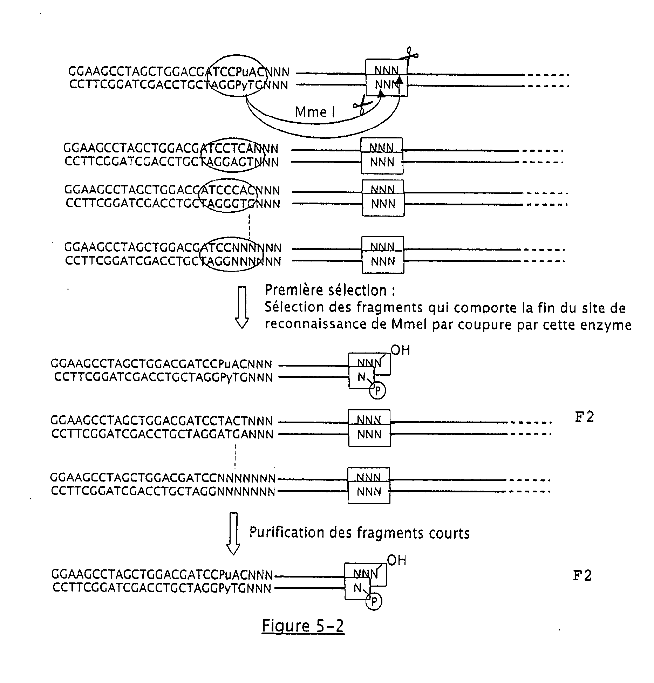

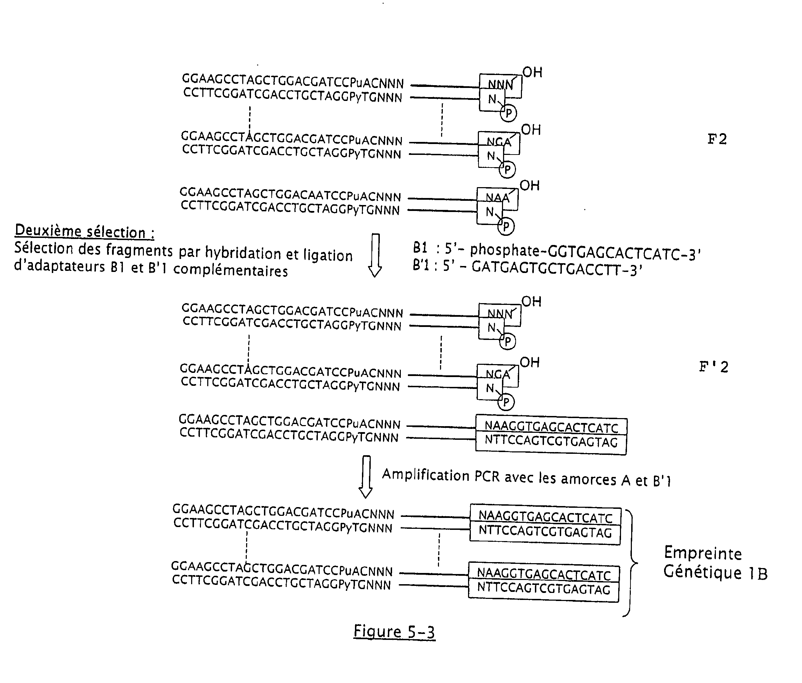

- les figures 5-1 à 5-3 illustrent un deuxième exemple des étape a) à f) du procédé selon l'invention : figure 5-1 : étapes a et b, figure 5-2 : étapes c et d, figure 5-3 : étapes e et f ;
- étape a) : les fragments d'ADN-double-brin sont générés par coupure avec *BamHI* qui reconnaît le site GGATCC,
- étape b) : l'adaptateur AA' (16/20 pb), comprend respectivement de 5' en 3': 15 paires de bases (zone 2 : GGAAGCCTAGCTGGA (SEQ ID NO:1) sur le brin A) et 1 pb de base non complémentaire du site *BamH I* (C sur le brin A), ainsi que 4 bases complémentaires du site *BamH I* (zone 1) incluant 2 bases de l'extrémité 5' du site *Mme I* (AG) et un résidu de phosphate, à l'extrémité 5' du brin A' (5'phosphate-GATC). L'ADN ligase permet la liaison de l'adaptateur aux extrémités cohésives des fragments *BamH I* par l'intermédiaire de liaisons phosphodiester, et
- étapes c) et d) : les fragments liés à l'adaptateur sont coupés à l'aide de l'enzyme *Mme I* (enzyme 2) de façon à générer des fragments courts (44/42 pb) à partir des fragments qui ont restauré le site *Mme I* (TCCPuAC) par ligation de l'adaptateur AA' avec un fragment dont l'extrémité 5' correspond à la séquence PuAC ; la sélection de 3 paires de bases spécifiques (PuAC) permet de diminuer le nombre de fragments d'un facteur 32 (2x4x4) par rapport à l'échantillon de départ.
- étape d) : les fragments courts obtenus à l'étape c) sont purifiés,
- étape e) : les fragments courts purifiés à l'étape d) sont liés à l'une de leur extrémité à un adaptateur B₁B₁' (14/16 pb) comprenant 2 bases complémentaires de l'extrémité dudit fragment (TT) à l'extrémité 3' du brin B'₁, et un groupement phosphate à l'extrémité 5' du brin B₁ ; la sélection de 2 bases spécifiques (AA) permet de diminuer le nombre de fragments d'un facteur 512 (2x4x4x16) par rapport à l'échantillon de départ et d'obtenir des fragments courts de 58 paires de bases comprenant 28 paires de bases spécifiques de l'ADN à analyser, lesquels fragments correspondent à 16 sous-groupes potentiels d'empreintes génétiques,
- étape f) : les fragments sélectionnés à l'étape e) sont amplifiés à l'aide d'amorces sens et anti-sens correspondant aux séquences complémentaires de respectivement les brins A et B'₁ des adaptateurs AA' et B₁B'₁.

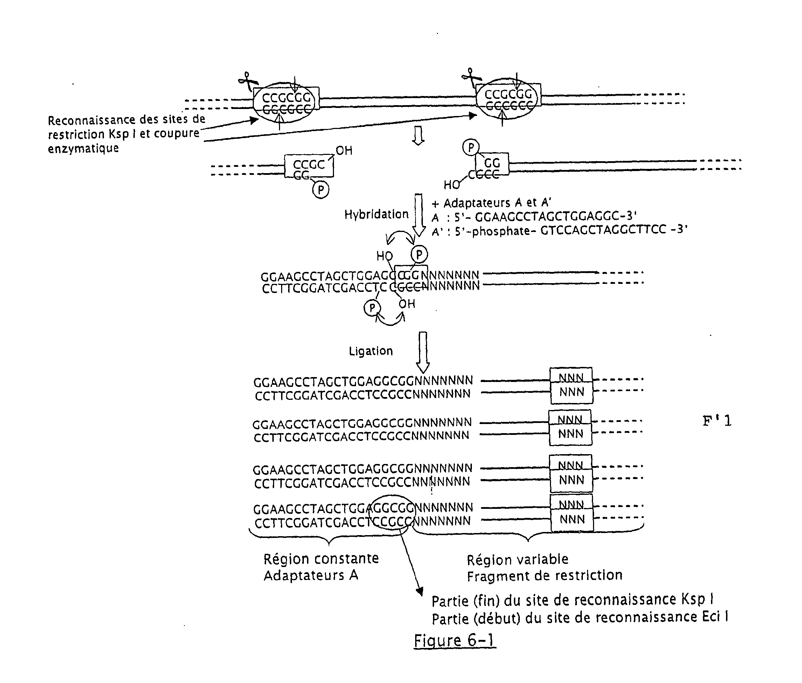

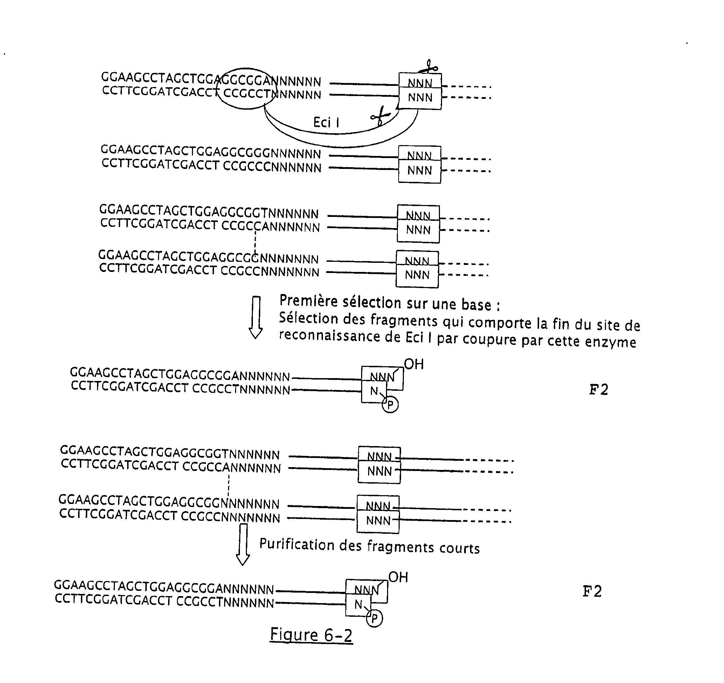

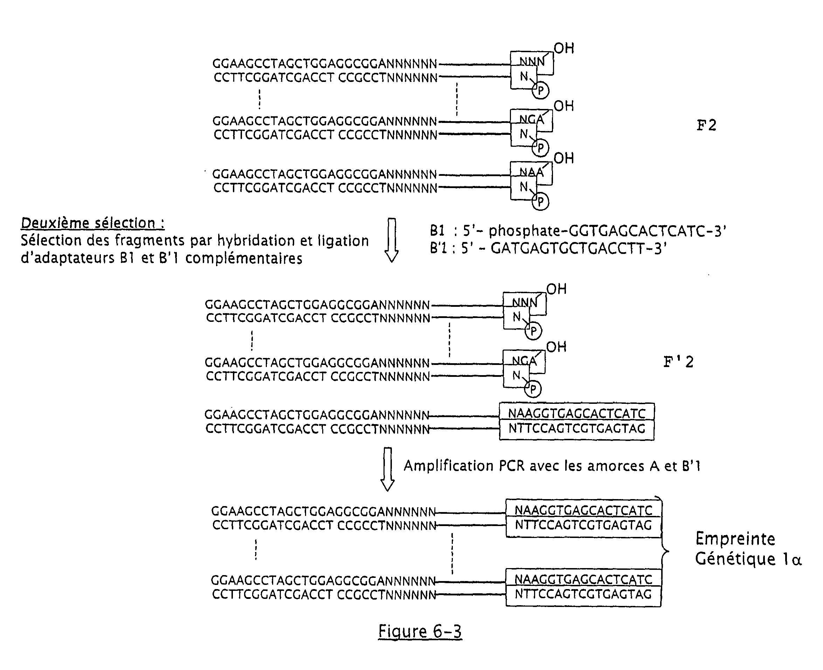

- les figures 6-1 à 6-3 illustrent un troisième exemple des étapes a) à f) du procédé selon l'invention : figure 6-1 : étapes a et b, figure 6-2 : étapes c et d, figure 6-3 : étapes e et f ;
- étape a) : les fragments d'ADN-double-brin sont générés par coupure avec *Ksp I* qui reconnaît le site CCGCGG,
- étape b) : l'adaptateur AA' (18/16 pb), comprend respectivement de 5' en 3': 15 paires de bases (zone 2 : GGAAGCCTAGCTGGA (SEQ ID NO:1) sur le brin A), ainsi qu'une paire de bases de la séquence 5' du site *Eci I* (G sur le brin A) et 2 bases complémentaires du site *Ksp I* (GC sur le brin A) et un résidu de phosphate, à l'extrémité 5'du brin A' ; ledit adaptateur incluant 3 bases de l'extrémité 5' du site *Eci I* (GGC). L'ADN ligase permet la liaison de l'adaptateur aux extrémités cohésives des fragments *Ksp I* par l'intermédiaire de liaisons phosphodiester, et
- étapes c) et d) : les fragments liés à l'adaptateur sont coupés à l'aide de l'enzyme *Eci I* (enzyme 2) de façon à générer des fragments courts à partir des fragments qui ont restauré le site *Eci I* (GGCGGA) par ligation de l'adaptateur AA' avec un fragment dont l'extrémité 5'correspond à la séquence A ; la sélection d'une paire de bases spécifiques permet de diminuer le nombre de fragments d'un facteur 4 par rapport à l'échantillon de départ.
- étape d) : les fragments courts obtenus à l'étape c) sont purifiés,
- étape e) : les fragments courts purifiés à l'étape d) sont liés à l'une de leur extrémité à un adaptateur B₁B₁' (14/16 pb) comprenant 2 bases complémentaires de l'extrémité dudit fragment (TT) à l'extrémité 3' du brin B'₁, et un groupement phosphate à l'extrémité 5' du brin B₁ ; la sélection de 2 bases spécifiques (AA) permet de diminuer le nombre de fragments d'un facteur 64 (4x16) par rapport à l'échantillon de départ et d'obtenir des fragments courts comprenant 28 paires de bases spécifiques de l'ADN à analyser, lesquels fragments correspondent à 16 sous-groupes potentiels d'empreintes génétiques,
- étape f) : les fragments sélectionnés à l'étape e) sont amplifiés à l'aide d'amorces sens et anti-sens correspondant aux séquences complémentaires de respectivement les brins A et B'₁ des adaptateurs AA' et B₁B'₁,
- la figure 7 illustre un exemple de mise en oeuvre du procédé selon l'invention à l'aide de deux enzymes E1 différentes (E1_{A} coupe fréquemment comme *Msp I* et *Taq^{α} I* et E1_{C} coupe rarement comme *Pst I*, *EcoR I*), de façon à réduire d'avantage la complexité de l'ADN à analyser, par introduction d'une sélection supplémentaire par la coupure par l'enzyme E1_{C}. Les séquences des sites de restriction sont indiquées dans le sens 5'→3' pour le brin positif. Les bases indiquées en gras restent sur le fragment d'intérêt après coupure. Les bases soulignées sont celles qui sont imposées par le couplage des enzymes E1_{A} et E2.
- la figure 8 illustre un exemple de mise en oeuvre du procédé selon l'invention à l'aide de deux enzymes E1 différentes, de façon à sélectionner un premier ensemble de fragments A=C puis une fraction de fragments courts F2 (étape c).

### Exemple 1 : Préparation de courts fragments d'ADN (cible ou sonde) selon le procédé de l'invention.

La préparation des acides nucléiques, les digestions enzymatiques, les ligations, les amplification PCR et la purification des fragments ainsi obtenus, ont été réalisées en utilisant les techniques classiques, selon les protocoles standards tels que ceux décrits dans Current Protocols in Molecular Biology (Frederick M. AUSUBEL, 2000, Wiley and son Inc, Library of Congress, USA).

L'ADN génomique a été extrait à partir de sang de bovin, à l'aide du kit *PAXgene Blood DNA* (référence 761133, QIAGEN), selon les instructions du fabricant.

Les adaptateurs et les amorces suivants ont été synthétisés par MWG BIOTECH :
-**adaptateur AA'**
   - brin A : 5'-GGAAGCCTAGCTGGAC-3' (SEQ ID NO:2)
   - brin A' : 5' -P-AATTCTCCAGCTAGGCTTCC-3' (SEQ ID NO:3)
- **adaptateur BB'**
   B:5'-P- GGTGAGCACTCATC-3' (SEQ ID NO:4)
   B' : 5'-GATGAGTGCTGACCTT-3' (SEQ ID NO: 5)
**- amorces**

On peut utiliser indifféremment le couple d'amorces 1 :
Sens : 5'-CCTTCGGATCGACCTG-3' (SEQ ID NO:6)
Anti-sens : 5'-CTACTCACGAGTGGAA-3' (SEQ ID NO:7)
ou le couple d'amorces 2 :
Sens : 5'-GGAAGCCTAGCTGGAC-3' (SEQ ID NO:2)
Anti-sens : 5'GATGAGTGCTGACCTT-3' (SEQ ID NO:5).
Le couple d'amorces 2 permet notamment de réutiliser une partie des séquences des adaptateurs.

Les fragments courts d'ADN (F2 et F'2) ont ensuite été préparés selon les étapes suivantes :

### 1) Gestion de l'ADN génomique par Eco RI et ligation des fragments à l'adaptateur AA' (étapes a et b)

L'ADN génomique purifié (5 µg) et l'adaptateur (5 µg) ont été incubés, 3 h à 37 °C, dans 40 µl de tampon 10 mM Tris-HCl, pH 7,5, 10 mM MgCl₂, 50 mM NaCl, 10 mM DTT, 1 mM EDTA, 1 mM ATP et 1 mg BSA, contenant 50 UI *d'EcoR I* et 2 UI de T4 ADN-ligase. Les fragments d'ADN liés à leurs extrémités à l'adaptateur AA' ainsi obtenus ont été purifiés par précipitation dans un mélange 1 : 4 (V/V) d'acétate d'ammonium 3M et d'éthanol.

### 2) Digestion des fragments par Mme I et sélection des fragments courts F2 (étapes c) et d))

Le culot a été remis en suspension dans 40 µl de tampon 50 mM acétate de potassium, 20 mM Tris-acétate, 10 mM acétate de magnésium, 1 mM DTT, pH 7,9 et incubé 1 h à 37 °C en présence de 5 UI de *Mme I*.

### 3) Purification de la fraction de fragments courts F2 (étape d)

L'enzyme a été éliminée à l'aide du kit Micropure-EZ (MILLIPORE), les sels ont ensuite été éliminés par filtration (Microcon YM3) puis l'ADN retenu sur le filtre YM3 a été élué et les fragments courts ont été purifiés par filtration (Microcon YM 30 ou YM 50, MILLIPORE); les fragments d'ADN de moins de 100 pb correspondant à l'éluat, les fragments de plus grande taille étant retenus sur le filtre.

### 4) Ligation des fragments courts F2 à l'adaptateur BB' (étape e)

Les fragments courts obtenus à l'étape d) et l'adaptateur BB' (3 µg) ont été incubés, 3 h à 37 °C, dans 40 µl de tampon 10 mM Tris-HCl, pH 7,5, 10 mM MgCl₂, 50 mM NaCl, 10 mM DTT, 1 mM EDTA, 1 mM ATP et 1 mg BSA, contenant 2 UI de T4 ADN-ligase.

### 5) Amplification des fragments courts liés à l'adaptateur BB' (F'2) (étape f)

Les fragments courts liés à l'adaptateur BB' obtenus à l'étape e) ont ensuite été amplifiés par PCR à l'aide du couple sens et anti-sens, correspondant aux séquences complémentaires des brins A et B' des adaptateurs AA' et BB', dans un volume réactionnel de 50 µl contenant : 1 ng de fragments d'ADN, 150 ng de chacune des amorces et 2 UI d'AmpliTaq GOLD® (PERKIN ELMER) dans un tampon 15 mM Tris HCl, pH 8,0, 10 mM KCl, 5 mM MgCl₂ et 200 µM dNTPs. L'amplification a été réalisée dans un thermocycleur, pendant 35 cycles comprenant : une étape de dénaturation à 94°C pendant 30 s, suivie d'une étape d'hybridation à 60°C pendant 30 s et d'une étape d'extension à 72 °C pendant 2 min. Les fragments amplifiés par PCR ont été purifiés à l'aide du kit *MinElute PCR Purification* (référence LSKG, QIAGEN), en suivant les instructions du fabricant.

L'enzyme, les sels et les dNTPs libres ont été éliminés par filtration sur Micropure-EZ (MILLIPORE) puis sur Microcon YM3 (MILLIPORE) et le produit d'amplification PCR retenu sur le filtre a ensuite été élué.

### Exemple 2 : Utilisation des ADN-cibles pour hybrider des sondes oligonucléotidiques

Les fragments courts d'ADN double-brin (ADN-cibles) obtenus à l'exemple 1 ont été convertis en ADN simple-brin par digestion, 30 min à 37 °C, dans un volume réactionnel de 40 µl contenant 3.10⁻³ UI de 5'-exonucléase dans un tampon 0,02 M citrate d'ammonium, pH 5. L'enzyme, les sels et les dNTPs libres ont été éliminés par filtration sur Micropure-EZ (MILLIPORE) puis sur Microcon YM3 (MILLIPORE) et l'ADN simple-brin retenu sur le filtre a ensuite été élué.

Un support en verre du type puce à ADN, sur lequel sont immobilisées des sondes oligonucléotidiques dont certaines sont complémentaires des fragments d'ADN-cible obtenus à l'exemple 1, a été préparé selon les techniques connues en elles-mêmes. Lesdits ADN-cibles ont ensuite été dilués dans du tampon d'hybridation (H7140, SIGMA) et 10 µl ont été déposés sur le support en verre, entre lame et lamelle. L'hybridation a ensuite été réalisée, en chambre humide dans un thermocycleur, dans les conditions suivantes : 80°C pendant 3 min, puis la température est abaissée à 50°C par palier de 0,1°C/s et enfin la température est maintenue à 50°C pendant 10 minutes. La réaction d'hybridation a ensuite été stoppée par dépôt des lames de verre sur la glace.

L'excès de fragments d'ADN-cible non-complémentaires des sondes a ensuite été éliminé par des lavages successifs : 30 s avec du SSC 2X (SIGMA, S6639), 30 s avec du SSC 2X additionné de 0,1 % SDS (L4522, SIGMA), et 30 s avec du SSC 0,2X, à + 4°C.

Les lames de verre ont ensuite été séchées et l'hybridation a été visualisée et analysée à l'aide d'un scanner (modèle Gentaq, GENOMIC SOLUTION).

### Exemple 3 : Préparation de courts fragments d'ADN à l'aide de deux enzymes E1 différentes E1_{A} et E1_{C}

L'ADN génomique est préparé comme décrit à l'exemple 1.

Les adaptateurs et les amorces suivants ont été synthétisés :
- **adaptateur AA'** (complémentaire du site *Taq^{α} I*)
   - brin A : 5'- GACGATGAGTCCTGAC-3' (SEQ ID NO : 8)
   - brin A': 5'-P- CGGTCAGGACTCATCGTC- 3'(SEQ ID NO: 9)
- **adaptateur CC'** (complémentaire du site *EcoR I*)
   - brin C : 5'P-AATTGGTACGCAGTCTAC-3'(SEQ ID NO : 10)
   - brin C' : 5'-GTAGACTGCGTACC-3' (SEQ ID NO : 11)
- **amorces**
   - amorce sens: 5'- Cy3-GACGATGAGTCCTGACCG-3' (SEQ ID NO : 12)
   - amorce anti-sens: 5'-biotine-GTAGACTGCGTACCAATT-3' (SEQ ID NO: 13)

Les fragments courts d'ADN (F2) ont ensuite été préparés selon les étapes suivantes :

### 1) Digestion de l'ADN génomique par Eco RI et Taq^{α} I et ligation des fragments aux adaptateurs AA' et CC' (étapes a et b)

L'ADN génomique purifié (5 µg) et chacun des adaptateurs (5 µg de AA' et 5 µg de CC') ont été incubés, 3 h à 37 °C, dans 40 µl de tampon 10 mM Tris-HCl, pH 7,5, 10 mM MgCl₂, 50 mM NaCl, 10 mM DTT, 1 mM EDTA, 1 mM ATP et 1 mg BSA, contenant 50 UI d'*EcoR I,* 50 UI de *Taq^{α} I* et 2 UI de T4 ADN-ligase.

### 2) Amplification des fragments F'1

Les fragments d'ADN F'1 liés en 5' à l'adaptateur AA' et en 3' à l'adaptateur CC' ont été amplifiés à l'aide des amorces sens et antisens (SEQ ID NO: 8 et 11) dans un mélange réactionnel de 50 µl final contenant 1 µl de fragments ligués, 2 UI de polymérase (AmpliTaq Gold, PERKIN-ELMER), 150 ng de chacune des amorces, 200 µM de chacun des dNTPs dans un tampon Tris-HCl, pH 8, 10 mM KCI, 5 mM MgCl₂. L'amplification est réalisée dans les conditions suivantes : 35 cycles comprenant une étape de dénaturation à 94°C pendant 30 s, une étape d'hybridation à 60°C pendant 30 s puis une étape d'élongation à 72 °C pendant 2 min.

### 3) Liaison des fragments F'1 (A=C) à des billes magnétiques fonctionnalisées

Les billes magnétiques fonctionnalisées par la streptavidine (DYNAL ou MOLECULAR PROBES ; 500 µg) remises en suspension, sont placées à proximité d'un aimant pour former un culot, puis le surnageant est éliminé. Les billes sont rincées deux fois dans 50 µl de tampon (2 M NaCl, 10 mM Tris-HCl pH 7,5, 1 mM EDTA) puis remises en suspension dans 100 µl de tampon (1 M NaCl, 5 mM Tris-HCl pH 7,5, 0,5 mM EDTA). Le produit de la réaction PCR (50 µl) est ajouté à la suspension de billes, puis le mélange est agité fortement puis incubé sous agitation, pendant 30 min à température ambiante. Le surnageant est ensuite éliminé par aimantation comme ci-dessus, et les billes sont rincées avec 100 µl de tampon 0,1X SSC, 0,1 % SDS.

### 3) Digestion des fragments F'1 (A=C) par BseR I et purification des fragments courts F2 (étapes c et d))

Le culot formé par les billes a été remis en suspension dans 40 µl de tampon de réaction de l'enzyme *BseR I* et incubé 3 h à 37 °C en présence de 5 UI de *BseR I.* Les fragments courts F2 qui ont été libérés dans le milieu réactionnel sont récupérés.

### 4) Hybridation des ADN-cibles

Un support en verre du type puce à ADN, sur lequel sont immobilisées des sondes oligonucléotidiques dont certaines sont complémentaires des fragments d'ADN-cible obtenus, a été préparé selon les techniques connues en elles-mêmes. Lesdits ADN-cibles ont ensuite été dénaturés pendant 3 min à 95°C, dilués dans du tampon d'hybridation (H7140, SIGMA) et 10 µl ont été déposés sur le support en verre, entre lame et lamelle. L'hybridation a ensuite été réalisée, en chambre humide, pendant 2 heures à 50 °C. La réaction d'hybridation a ensuite été stoppée par dépôt des lames de verre sur la glace.

L'excès de fragments d'ADN-cible non-complémentaires des sondes a ensuite été éliminé par des lavages successifs : 30 s avec du SSC 2X (SIGMA, S6639), 30 s avec du SSC 2X additionné de 0,1 % SDS (L4522, SIGMA), et 30 s avec du SSC 0,2X, à + 4°C.

Les lames de verre ont ensuite été séchées et l'hybridation a été visualisée et analysée à l'aide d'un scanner (modèle Gentaq, GENOMIC SOLUTION).

## Revendications

1. Procédé de préparation de fragments d'ADN, à partir d'un échantillon d'acides nucléiques à analyser, lequel procédé est **caractérisé en ce qu'**il comprend la fragmentation sélective desdits acides nucléiques à l'aide d'au moins les étapes suivantes :
I. Une première sélection de fragments courts comprenant :
a) la préparation de premiers fragments d'ADN double-brin F1 à l'aide d'au moins une enzyme de restriction E1, apte à fragmenter de manière aléatoire l'échantillon d'acides nucléiques à analyser, en générant lesdits fragments F1 d'ADN aux extrémités franches ou cohésives,
b) l'obtention de fragments d'ADN F'1 par ligation des extrémités desdits fragments d'ADN F1 obtenus à l'étape a) à au moins un adaptateur AA', de manière à former un motif situé à la jonction de l'extrémité 3' dudit adaptateur et de l'extrémité 5' desdits fragments F1, tel que la séquence dudit motif qui correspond à la fin du site de reconnaissance de l'enzyme de restriction E1, contient les premières paires de bases du site de reconnaissance d'une enzyme de restriction E2 dont le site de coupure est situé en aval dudit site de reconnaissance,
c) la sélection d'une fraction de fragments courts F2 par coupure, à l'aide de ladite enzyme de restriction E2, de la fraction de fragments F'1 d'ADN obtenus en b) qui ont restauré la totalité du site de reconnaissance de ladite enzyme de restriction E2 par ligation de leurs extrémités audit adaptateur AA', et
d) la purification par tout moyen approprié de ladite fraction de fragments courts F2,
et éventuellement,
II. Une deuxième sélection d'un ou plusieurs sous-ensembles de fragments à partir de la fraction de fragments courts F2 obtenue à l'étape d), conformément aux étapes suivantes :
e) la ligation de l'extrémité libre (non liée à l'adaptateur AA') de fragments courts F2 obtenus en d) à au moins un second adaptateur complémentaire BB' (obtention de fragments F'2), et
f) l'amplification des fragments courts F'2 liés auxdits adaptateurs (AA' et BB'), à l'aide d'au moins un couple d'amorces appropriées, l'une au moins étant éventuellement marquée à son extrémité 5', de façon à sélectionner au moins un sous-ensemble de fragments courts F'2, à partir de la fraction de fragments courts F2 obtenue en d).

2. Procédé selon la revendication 1, **caractérisé en ce que** l'étape a) est réalisée avec deux enzymes de restriction E1 différentes, E1_{A} et E1_{C}, telles que :
- l'une au moins génère des extrémités cohésives, distinctes de celles éventuellement générées par l'autre enzyme de restriction, et
- l'extrémité 3' du site de restriction de E1_{1A} est celle du motif tel que défini à l'étape b).

3. Procédé selon la revendication 2, **caractérisé en ce que** l'une des enzymes coupe fréquemment et l'autre rarement.

4. Procédé selon la revendication 3, **caractérisé en ce que** :
- l'enzyme qui coupe fréquemment est l'enzyme E1_{A}, laquelle enzyme E1_{A} génère au moins une extrémité d'un fragment F1, qui se lie à l'adaptateur AA' à l'étape b), et
- l'enzyme E1_{C} qui coupe rarement, génère au moins une extrémité d'un fragment F1, qui se lie à l'étape b), à un second adaptateur CC' qui est différent de l'adaptateur AA'.

5. Procédé selon l'une quelconque des revendications 1 à 4, **caractérisé en ce que** les étapes a) et b) sont réalisées simultanément.

6. Procédé selon l'une quelconque des revendications 1 à 5, **caractérisé en ce qu'**il comprend une étape additionnelle de purification des fragments inférieurs à 1000 pb, préalablement à l'étape b) de ligation.

7. Procédé selon l'une quelconque des revendications 1 à 6, **caractérisé en ce que** l'adaptateur AA' tel que défini à l'étape b) comprend à l'extrémité 3' du brin A et/ou 5' du brin A', une zone 1 d'environ 1 à 8 bases ou paires de bases qui est identique ou complémentaire- en totalité ou en partie- au site de restriction de l'enzyme E1, laquelle zone 1 est choisie de manière à reconstituer la séquence des premières bases ou paires de bases du site de reconnaissance de l'enzyme de restriction E2, par ligation dudit adaptateur AA' aux extrémités desdits fragments d'ADN obtenus en a).

8. Procédé selon la revendication 7, **caractérisé en ce que** ladite zone 1 inclut un ou plusieurs misappariements avec la séquence dudit site de coupure de l'enzyme de restriction E1.

9. Procédé selon l'une quelconque des revendications 1 à 8, **caractérisé en ce que** l'adaptateur tel que défini à l'étape b) comprend en amont de la zone 1, une zone 2 d'au moins 6 paires de bases.

10. Procédé selon l'une quelconque des revendications 1 à 9, **caractérisé en ce que** l'adaptateur tel que défini à l'étape b) comprend au moins une base située entre la zone 1 et la zone 2, différente de celle qui dans le site de coupure de l'enzyme de restriction E1, est immédiatement adjacente à la séquence complémentaire correspondant à la zone 1.

11. Procédé selon l'une quelconque des revendications 1 à 10, **caractérisé en ce que** l'adaptateur tel que défini à l'étape b) comprend un résidu de phosphate lié de façon covalente à l'extrémité 5' du brin A'.

12. Procédé selon l'une quelconque des revendications 1 à 11, **caractérisé en ce que** lorsque ledit procédé consiste en une seule sélection de fragments courts selon les étapes a) à d), il comprend au moins une étape additionnelle b'), c') et/ou d'), d'amplification des fragments F'1 ou F2 à l'aide d'un couple d'amorces appropriées, de préférence un couple d'amorces marquées.

13. Procédé selon l'une quelconque des revendications 1 à 12, **caractérisé en ce que** l'adaptateur AA' tel que défini à l'étape b) est lié à l'extrémité 5' de son brin A à un marqueur approprié, notamment un marqueur qui permet la détection d'hybrides d'acides nucléiques ou un marqueur qui peut se fixer sur un support solide fonctionnalisé.

14. Procédé selon l'une quelconque des revendications 2 à 13, **caractérisé en ce que** l'extrémité 5' du brin C' de l'adaptateur CC' est liée à un marqueur, apte à se fixer sur un support solide fonctionnalisé, lequel marqueur est différent du marqueur lié à l'extrémité 5' du brin A de l'adaptateur AA'.

15. Procédé selon l'une quelconque des revendications 12 à 14, **caractérisé en ce que** les fragments F'1 obtenus à l'étape b) ou b') sont mis en contact avec ledit support fonctionnalisé préalablement à l'étape c) de coupure, et la fraction de fragments courts F2 de l'étape d) correspond, à la fraction de fragments qui est, soit retenue sur ledit support (adaptateur AA' lié au marqueur qui se fixe sur le support), soit libre (adaptateur CC' lié au marqueur qui se fixe sur le support).

16. Procédé selon l'une quelconque des revendications 1 à 11 et 13 à 15, **caractérisé en ce qu'**il comprend à l'étape e), la ligation de plusieurs adaptateurs complémentaires différents (B₁B₁', B₂B₂'..), chacun comprenant, à l'extrémité 5' du brin B ou à l'extrémité 3' du brin B', une séquence spécifique de 1 à 10 bases.

17. Procédé selon l'une quelconque des revendications 1 à 11 et 13 à 16, **caractérisé en ce** ledit adaptateur BB' tel que défini à l'étape e) comprend un résidu de phosphate lié de façon covalente à l'extrémité 5' du brin B.

18. Procédé selon l'une quelconque des revendications 1 à 11 et 13 à 17, **caractérisé en ce que** l'une des amorces telles que définies à l'étape f) est liée à son extrémité 5' à un marqueur approprié.

19. Procédé selon l'une quelconque des revendications 1 à 18, **caractérisé en ce qu'**il comprend une étape additionnelle d") ou g) d'obtention, par tout moyen approprié, de fragments simple-brin à partir des fragments courts F2 obtenus à l'étape d) ou d') ou bien des fragments courts F'2 obtenus à l'étape f).

20. Procédé selon l'une quelconque des revendications 1 à 19, **caractérisé en ce qu'**il comprend une étape additionnelle de purification, par tout moyen approprié, des produits d'amplification obtenus à l'étape b'), c'), d') ou f) ou des fragments simple brin obtenus à l'étape d") ou g).

21. Ensemble de fragments courts d'ADN, représentant un marqueur génétique susceptible d'être obtenu par le procédé selon l'une quelconque des revendications 1 à 20, **caractérisé en ce qu'**il comprend des fragments d'ADN de moins de 100 bases ou paires de bases, lesquels fragments comprenant au moins une séquence spécifique constituée par un fragment de séquence génomique ou de séquence d'ADNc bordé respectivement par le site de reconnaissance et le site de clivage d'une enzyme de restriction E2 dont le site de clivage est situé en aval dudit site de reconnaissance, telle que les premières paires de base du site de reconnaissance de ladite enzyme de restriction E2 correspondent à la fin du site de reconnaissance d'une enzyme de restriction E1 telle que définie à la revendication 1 et les dernières paires de bases du site de reconnaissance de ladite enzyme E2 correspondent à l'extrémité 5' de ladite séquence spécifique, ledit marqueur incluant à chaque extrémité au moins 6 bases ou 6 paires de bases de séquence non-spécifique.

22. Ensemble de fragments courts d'ADN selon la revendication 21, **caractérisé en ce qu'**il s'agit d'un ensemble de fragments simple-brin.

23. Ensemble de fragments courts d'ADN selon la revendication 21 ou la revendication 22, **caractérisé en ce que** l'une des extrémités 5' desdits fragments est liée à un marqueur approprié.

24. Puce à ADN, **caractérisée en ce qu'**elle comprend un ensemble de fragments d'ADN selon l'une quelconque des revendications 21 à 23.

25. Utilisation d'un ensemble de fragments d'ADN selon l'une quelconque des revendications 21 à 23 comme marqueur génétique.

26. Méthode d'hybridation d'acides nucléiques, **caractérisée en ce qu'**elle met en oeuvre un ensemble de fragments d'ADN selon l'une quelconque des revendications 21 à 23 ou une puce à ADN selon la revendication 24.

27. Kit pour la mise en oeuvre d'une méthode d'hybridation, **caractérisé en ce qu'**il comprend au moins un ensemble de fragments d'ADN selon l'une quelconque des revendications 21 à 23 ou une puce à ADN selon la revendication 24.

28. Kit selon la revendication 27, **caractérisé en ce qu'**il comprend également une sonde oligonucléotidique complémentaire desdits fragments d'ADN.

29. Utilisation d'un adaptateur AA' tel que défini à l'une quelconque des revendications 7 à 11 en combinaison avec une enzyme de restriction E2 telle que définie à la revendication 1, pour la préparation de marqueurs génétiques tels que définis à l'une quelconque des revendications 21 à 23.

30. Kit pour la mise en oeuvre du procédé selon l'une quelconque des revendications 1 à 20, **caractérisé en ce qu'**il comprend au moins un adaptateur AA' tel que défini tel que défini à l'une quelconque des revendications 7 à 11 et une enzyme de restriction E2 telle que définie à la revendication 1.

31. Kit selon la revendication 30, **caractérisé en ce qu'**il comprend également au moins un adaptateur BB' tel que défini à la revendication 1, 16 ou 17 et un couple d'amorces, telles que définies à la revendication 1 ou 18.

## Claims

1. Method for preparing DNA fragments from a sample of nucleic acids to be analysed, **characterised in that** it comprises the selective fragmentation of said nucleic acids by means of at least the following steps:
I. A first selection of short fragments comprising:
a) the preparation of first double-stranded DNA fragments F1 using at least one restriction enzyme E1 which can randomly fragment the sample of nucleic acids to be analysed, generating said DNA fragments F1 with blunt or cohesive ends,
b) obtaining DNA fragments F'1 by ligating the ends of said DNA fragments F1 obtained in step a) to at least one adapter AA' so as to form a motif located at the junction of the 3' end of said adapter and the 5' end of said fragments F1 such that the sequence of said motif which corresponds to the end of the recognition site of the restriction enzyme E1 contains the first base pairs of the recognition site of a restriction enzyme E2, of which the cutting site is located downstream of said recognition site,
c) the selection of a fraction of short fragments F2 through cutting, using said restriction enzyme E2, of the fraction of DNA fragments F'1 obtained in b) which have restored the whole of the recognition site of said restriction enzyme E2 through ligation of their ends to said adapter AA', and
d) the purification by any appropriate means of said fraction of short fragments F2,
and optionally
II. a second selection of one or more subsets of fragments from the fraction of short fragments F2 obtained in step d) in accordance with the following steps:
e) the ligation of the free end (not linked to the adapter AA') of short fragments F2 obtained in d) to at least a second complementary adapter BB' (obtaining of fragments F'2), and
f) the amplification of the short fragments F'2 linked to said adapters (AA' and BB') using at least one pair of appropriate primers, at least one being possibly labelled at its 5' end, so as to select at least one subset of short fragments F'2 from the fraction of short fragments F2 obtained in d).

2. Method according to claim 1, **characterised in that** step a) is realised with two different E1 restriction enzymes, E1_{A} and E1_{C}, such that
- at least one generates cohesive ends, different from those optionally generated by the other restriction enzyme, and
- the 3' end of the restriction site of E1_{1A} is that of the motif as defined in step b).

3. Method according to claim 2, **characterised in that** one of the enzymes cuts frequently and the other rarely.

4. Method according to claim 3, **characterised in that**:
- the enzyme which cuts frequently is the enzyme E1_{A}, which enzyme E1_{A} generates at least one end of a fragment F1 which binds in step b) to the adapter AA', and
- the enzyme E1_{C} which cuts rarely generates at least one end of a fragment F1 which binds in step b) to a second adapter CC' which is different from the adapter AA'.

5. Method according to any one of the claims 1 to 4, **characterised in that** the steps a) and b) are realised simultaneously.

6. Method according to any one of the claims 1 to 5, **characterised in that** it comprises an additional step of purification of the fragments of less than 1000 pb prior to the ligation step b).

7. Method according to any one of the claims 1 to 6, **characterised in that** the adapter AA' as defined in step b) comprises, at the 3' end of the strand A and / or 5' of the strand A', a zone 1 of approximately 1 to 8 bases or base pairs which is identical or complementary - in full or in part - to the restriction site of the enzyme E1, which zone 1 is selected in such a way as to reconstitute the sequence of the first bases or base pairs of the recognition site of the restriction enzyme E2, through ligation of said adapter AA' to the ends of said DNA fragments obtained in a).

8. Method according to claim 7, **characterised in that** said zone 1 includes one or more mismatches with the sequence of said cutting site of the restriction enzyme E1.

9. Method according to any one of the claims 1 to 8, **characterised in that** the adapter as defined in step b) comprises a zone 2 of at least 6 base pairs upstream of the zone 1.

10. Method according to any one of the claims 1 to 9, **characterised in that** the adapter as defined in step b) comprises at least one base located between the zone 1 and the zone 2, different from that which in the cutting site of the restriction enzyme E1 is immediately adjacent to the complementary sequence corresponding to the zone 1.

11. Method according to any one of the claims 1 to 10, **characterised in that** the adapter as defined in step b) comprises a phosphate residue covalently linked to the 5' end of the strand A'.

12. Method according to any one of the claims 1 to 11, **characterised in that** when said method consists of a single selection of short fragments according to steps a) to d), it comprises at least one additional step b'), c') and / or d') of amplification of the fragments F'1 or F2 using an appropriate pair of primers, preferably a pair of labelled primers.

13. Method according to any one of the claims 1 to 12, **characterised in that** the adapter AA' as defined in step b) is linked at the 5' end of its strand A to an appropriate label, particularly a label for detecting nucleic acid hybrids or a label attachable to a functionalised solid support.

14. Method according to any one of the claims 2 to 13, **characterised in that** the 5' end of the strand C' of the adapter CC' is linked to a label which is attachable to a functionalised solid support, said label being different from the label linked to the 5' end of the strand A of the adapter AA'.

15. Method according to any one of the claims 12 to 14, **characterised in that** the fragments F'1 obtained in step b) or b') are brought into contact with said functionalised support prior to the cutting step c) and the fraction of short fragments F2 of the step d) corresponds to the fraction of fragments which is either retained on said support (adapter AA' linked to the label which attaches to the support) or free (adapter CC' linked to the label that attaches to the support).

16. Method according to any one of the claims 1 to 11 and 13 to 15, **characterised in that** it comprises in step e) the ligation of several different complementary adapters (B₁B₁', B₂B₂'), each comprising at the 5' end of the strand B or at the 3' end of the strand B' a specific sequence of 1 to 10 bases.

17. Method according to any one of the claims 1 to 11 and 13 to 16, **characterised in that** said adapter BB' as defined in step e) comprises a phosphate residue linked covalently to the 5' end of the strand B.

18. Method according to any one of the claims 1 to 11 and 13 to 17, **characterised in that** one of the primers as defined in step f) is linked at its 5' end to an appropriate label.

19. Method according to any one of the claims 1 to 18, **characterised in that** it comprises an additional step d") or g) of obtaining by any appropriate means single-stranded fragments from the short fragments F2 obtained in step d) or d') or from the short fragments F'2 obtained in step f).

20. Method according to any one of the claims 1 to 19, **characterised in that** it comprises an additional step of purifying by any appropriate means the amplification products obtained in step b'), c'), d') or f) or the single-stranded fragments obtained in step d") or g).

21. Set of short DNA fragments representing a genetic marker that can be obtained by the method according to any one of the claims 1 to 20, **characterised in that** it comprises DNA fragments of less than 100 bases or base pairs, said fragments comprising at least one specific sequence constituted by a fragment of genomic sequence or cDNA sequence bordered respectively by the recognition site and the cleavage site of a restriction enzyme E2, of which the cleavage site is located downstream of said recognition site, such that the first base pairs of the recognition site of said restriction enzyme E2 correspond to the end of the recognition site of a restriction enzyme E1 such as defined in claim 1 and the last base pairs of the recognition site of said enzyme E2 correspond to the 5' end of said specific sequence, said marker including at each end at least 6 bases or 6 base pairs of non-specific sequence.

22. Set of short DNA fragments according to claim 21, **characterised in that** it is a set of single-stranded fragments.

23. Set of short DNA fragments according to claim 21 or claim 22, **characterised in that** one of the 5' ends of said fragments is linked to an appropriate label.

24. DNA chip, **characterised in that** it comprises a set of DNA fragments according to any one of the claims 21 to 23.

25. Use of a set of DNA fragments according to any one of the claims 21 to 23 as a genetic marker.

26. Method of hybridisation of nucleic acids, **characterised in that** it implements a set of DNA fragments according to any one of the claims 1 to 23 or a DNA chip according to claim 24.

27. Kit for carrying out a hybridisation method, **characterised in that** it comprises at least one set of DNA fragments according to any one of the claims 21 to 23 or a DNA chip according to claim 24.

28. Kit according to claim 27, **characterised in that** it also comprises a complementary oligonucleotidic probe of said DNA fragments.

29. Use of an adapter AA' as defined in any one of the claims 7 to 11 in combination with a restriction enzyme E2 as defined in claim 1 for the preparation of genetic markers as defined in any one of the claims 21 to 23.

30. Kit for carrying out the method according to any one of the claims 1 to 20, **characterised in that** it comprises at least one adapter AA' as defined in any one of the claims 7 to 11 and a restriction enzyme E2 as defined in claim 1.

31. Kit according to claim 30, **characterised in that** it also comprises at least one adapter BB' as defined in claim 1, 16 or 17 and a pair of primers as defined in claims 1 or 18.

## Patentansprüche

1. Verfahren zur Herstellung von DNA-Fragmenten aus einer zu analysierenden Probe von Nucleinsäuren, **dadurch gekennzeichnet, dass** es die selektive Fragmentierung der Nucleinsäuren mit Hilfe wenigstens der folgenden Stufen umfasst:
I. eine erste Selektion von kurzen Fragmenten, umfassend:
a) die Herstellung von ersten Fragmenten doppelsträngiger DNA F1 mit Hilfe wenigstens eines Restriktionsenzyms E1, das fähig ist, die zu analysierende Probe von Nucleinsäuren in zufälliger Art unter Bildung der DNA-Fragmente F1 mit glatten oder kohäsiven Enden zu fragmentieren,
b) den Erhalt von DNA-Fragmenten F'1 durch Ligation der Enden der DNA-Fragmente F1, die in Stufe a) erhalten wurden, an wenigstens einen Adapter AA' derart, dass ein Motiv gebildet wird, dass sich an der Verbindung des 3' -Endes des Adapters und des 5'-Endes der Fragmente F1 befindet, wobei die Sequenz des Motivs, die dem Ende der Erkennungsstelle des Restriktionsenzyms E1 entspricht, die ersten Basenpaare der Erkennungsstelle eines Restriktionsenzyms E2 enthält, dessen Schnittstelle stromabwärts der Erkennungsstelle gelegen ist,
c) die Selektion einer Fraktion von kurzen Fragmenten F2 durch Schneiden mit Hilfe des Restriktionsenzyms E2 der Fraktion von DNA-Fragmenten F'1, die in b) erhalten wurden, die die Gesamtheit der Erkennungsstelle des Restriktionsenzyms E2 durch Ligation ihrer Enden an den Adapter AA' wieder hergestellt haben, und
d) die Reinigung der Fraktion von kurzen Fragmenten F2 durch jedes geeignete Mittel, und
gegebenenfalls
II. eine zweite Selektion einer Untergruppe oder mehrerer Untergruppen von Fragmenten aus der Fraktion von kurzen Fragmenten F2, die in Stufe d) erhalten wurde, nach den folgenden Stufen:
e) die Ligation des freien Endes (nicht an den Adapter AA' gebunden) von kurzen Fragmenten F2, die in d) erhalten wurden, an wenigstens einen zweiten komplementären Adapter BB' (Erhalt von Fragmenten F'2) und
f) die Amplifikation der kurzen Fragmente F'2, die an die Adapter (AA' und BB') gebunden sind, mit Hilfe wenigstens eines Paares geeigneter Primer, wobei der eine wenigstens gegebenenfalls an seinem 5'-Ende markiert ist, um wenigstens eine Untergruppe von kurzen Fragmenten F'2 aus der Fraktion von kurzen Fragmenten F2, die in d) erhalten wurde, zu selektionieren.

2. Verfahren nach Anspruch 1, **dadurch gekennzeichnet, dass** die Stufe a) mit zwei verschiedenen Restriktionsenzymen E1, E1_{A} und E1_{C}, durchgeführt wird, wobei
- wenigstens das eine kohäsive Enden erzeugt, die verschieden von denen sind, die gegebenenfalls durch das andere Restriktionsenzym erzeugt werden, und
- das 3'-Ende der Restriktionsstelle von E1_{1A} diejenige des Motivs ist, wie es in Stufe b) definiert ist.

3. Verfahren nach Anspruch 2, **dadurch gekennzeichnet, dass** das eine der Enzyme häufig schneidet und das andere selten.

4. Verfahren nach Anspruch 3, **dadurch gekennzeichnet, dass**:
- das Enzym, das häufig schneidet, das Enzym E1_{A} ist, wobei das Enzym E1_{A} wenigstens ein Ende eines Fragments F1 erzeugt, das in der Stufe b) an den Adapter AA' bindet und
- das Enzym E1_{C}, das selten schneidet, wenigstens ein Ende eines Fragmentes F1 erzeugt, das in der Stufe b) an einen zweiten Adapter CC' bindet, der von dem Adapter AA' verschieden ist.

5. Verfahren nach einem der Ansprüche 1 bis 4, **dadurch gekennzeichnet, dass** die Stufen a) und b) gleichzeitig durchgeführt werden.

6. Verfahren nach einem der Ansprüche 1 bis 5, **dadurch gekennzeichnet, dass** es eine zusätzliche Stufe der Reinigung der Fragmente unter 1.000 bp vor der Stufe b) der Ligation umfasst.

7. Verfahren nach einem der Ansprüche 1 bis 6, **dadurch gekennzeichnet, dass** der Adapter AA', wie er in Stufe b) definiert wurde, am 3'-Ende des Strangs A und/oder 5'-Ende des Strangs A' eine Zone 1 mit etwa 1 bis 8 Basen oder Basenpaaren umfasst, die ganz oder teilweise zur Restriktionsstelle des Enzyms identisch oder komplementär ist, wobei die Zone 1 so gewählt ist, dass sie die Sequenz der ersten Basen oder Basenpaare der Erkennungsstelle des Restriktionsenzyms E2 durch Ligation des Adapters AA' an die Enden der in a) erhaltenen DNA-Fragmente neu konstruiert.

8. Verfahren nach Anspruch 7, **dadurch gekennzeichnet, dass** die Zone 1 eine oder mehrere Fehlpaarungen mit der Sequenz der Schnittstelle des Restriktionsenzyms E1 einschließt.

9. Verfahren nach einem der Ansprüche 1 bis 8, **dadurch gekennzeichnet, dass** der Adapter, wie er in Stufe b) definiert ist, stromaufwärts der Zone 1 eine Zone 2 mit wenigstens 6 Basenpaaren umfasst.

10. Verfahren nach einem der Ansprüche 1 bis 9, **dadurch gekennzeichnet, dass** der Adapter, wie er in Stufe b) definiert ist, wenigstens eine Base umfasst, die sich zwischen der Zone 1 und der Zone 2 befindet, die sich von der unterscheidet, die in der Schnittstelle des Restriktionsenzyms E1 umittelbar angrenzend an die komplementäre Sequenz ist, die Zone 1 entspricht.

11. Verfahren nach einem der Ansprüche 1 bis 10, **dadurch gekennzeichnet, dass** der Adapter, wie er in Stufe b) definiert ist, einen Phosphatrest umfasst, der in kovalenter Art an das 5'-Ende des Strangs A' gebunden ist.

12. Verfahren nach einem der Ansprüche 1 bis 11, **dadurch gekennzeichnet, dass**, wenn das genannte Verfahren in einer alleinigen Selektion von kurzen Fragmenten gemäß den Stufen a) bis d) besteht, es wenigstens eine zusätzliche Stufe b'), c') und/oder d') der Amplifikation der Fragmente F'1 oder F2 mit Hilfe eines Paares geeigneter Primer, vorzugsweise eines Paares markierter Primer, umfasst.

13. Verfahren nach einem der Ansprüche 1 bis 12, **dadurch gekennzeichnet, dass** der Adapter AA' , wie er in Stufe b) definiert ist, am 5'-Ende seines Strangs A an einen geeigneten Marker, insbesondere einen Marker, der die Detektion von Hybriden von Nucleinsäuren erlaubt, oder einen Marker, der sich an einem funktionalisierten festen Träger fixieren kann, gebunden ist.

14. Verfahren nach einem der Ansprüche 2 bis 13, **dadurch gekennzeichnet, dass** das 5' -Ende des Strangs C' des Adapters CC' an einen Marker gebunden ist, der fähig ist, sich an einem funktionalisierten festen Träger zu fixieren, wobei der Marker von dem Marker, der am 5'-Ende des Strangs A des Adapters AA' gebunden ist, verschieden ist.

15. Verfahren nach einem der Ansprüche 12 bis 14, **dadurch gekennzeichnet, dass** die Fragmente F'1, die in der Stufe b) oder b') erhalten wurden, mit dem vorher funktionalisierten Träger in der Stufe c) des Schneidens in Kontakt gebracht werden und die Fraktion von kurzen Fragmenten F2 der Stufe d) der Fraktion von Fragmenten entspricht, die entweder auf dem festen Träger zurückgehalten wird (Adapter AA', gebunden an den Marker, der sich auf dem Träger fixiert) oder die frei ist (Adapter CC', an den Marker gebunden, der sich auf dem Träger fixiert).

16. Verfahren nach einem der Ansprüche 1 bis 11 und 13 bis 15, **dadurch gekennzeichnet, dass** es in der Stufe e) die Ligation von mehreren verschiedenen komplementären Adaptern (B1B1', B2B2' ...) umfasst, von denen jeder am 5'-Ende des Strangs B oder am 3'-Ende des Strangs B' eine spezifische Sequenz mit 1 bis 10 Basen umfasst.

17. Verfahren nach einem der Ansprüche 1 bis 11 und 13 bis 16, **dadurch gekennzeichnet, dass** der Adapter BB', wie er in Stufe e) definiert ist, einen Phosphatrest umfasst, der in kovalenter Art an das 5'-Ende des Strangs B gebunden ist.

18. Verfahren nach einem der Ansprüche 1 bis 11 und 13 bis 17, **dadurch gekennzeichnet, dass** einer der Primer, wie sie in Stufe f) definiert sind, an seinem 5'-Ende an einen geeigneten Marker gebunden ist.

19. Verfahren nach einem der Ansprüche 1 bis 18, **dadurch gekennzeichnet, dass** es eine zusätzliche Stufe d") oder g) des Erhalts durch jedes geeignete Mittel von Einzelstrangfragmenten aus den kurzen Fragmenten F2, die in der Stufe d) oder d') erhalten wurden, oder auch aus den kurzen Fragmenten F'2, die in Stufe f) erhalten wurden, umfasst.

20. Verfahren nach einem der Ansprüche 1 bis 19, **dadurch gekennzeichnet, dass** es eine zusätzliche Stufe der Reinigung der Amplifikationsprodukte, die in der Stufe b'), c'), d') oder f) erhalten wurden, oder der Einzelstrangfragmente, die in der Stufe d") oder g) erhalten wurden, durch jedes geeignete Mittel umfasst.

21. Gesamtheit von kurzen DNA-Fragmenten, die einen genetischen Marker darstellt, der durch ein Verfahren nach einem der Ansprüche 1 bis 20 erhältlich ist, **dadurch gekennzeichnet, dass** sie DNA-Fragmente mit weniger als 100 Basenpaaren oder Basen umfasst, wobei die Fragmente wenigstens eine spezifische Sequenz umfassen, die durch ein Fragment einer genomischen Sequenz oder cDNA-Sequenz gebildet wird, welche von der Erkennungsstelle und der Spaltstelle eines Restriktionsenzyms E2 flankiert ist, dessen Spaltungsstelle stromabwärts der Erkennungsstelle liegt, wobei die ersten Basenpaare der Erkennungsstelle des Restriktionsenzyms E2 dem Ende der Erkennungsstelle eines Restriktionsenzyms E1, wie es in Anspruch 1 definiert ist, entsprechen und die letzten Basenpaare der Erkennungsstelle des Enzyms E2 dem 5'-Ende der spezifischen Sequenz entsprechen, wobei der Marker an jedem Ende wenigstens 6 Basen oder 6 Basenpaare mit nicht spezifischer Sequenz enthält.

22. Gesamtheit kurzer DNA-Fragmente nach Anspruch 21, **dadurch gekennzeichnet, dass** es sich um eine Gesamtheit von Einzelstrangfragmenten handelt.

23. Gesamtheit von kurzen DNA-Fragmenten nach Anspruch 21 oder Anspruch 22, **dadurch gekennzeichnet, dass** das eine der 5'-Enden der Fragmente an einen geeigneten Marker gebunden ist.

24. DNA-Chip, **dadurch gekennzeichnet, dass** er eine Gesamtheit von DNA-Fragmenten nach einem der Ansprüche 21 bis 23 umfasst.

25. Verwendung einer Gesamtheit von DNA-Fragmenten nach einem der Ansprüche 21 bis 23 als genetischer Marker.

26. Verfahren zur Hybridisierung von Nucleinsäuren, **dadurch gekennzeichnet, dass** es eine Gesamtheit von DNA-Fragmenten nach einem der Ansprüche 21 bis 23 oder einen DNA-Chip nach Anspruch 24 verwendet.

27. Kit zur Durchführung eines Verfahrens zur Hybridisierung, **dadurch gekennzeichnet, dass** er wenigstens eine Gesamtheit von DNA-Fragmenten nach einem der Ansprüche 21 bis 23 oder einen DNA-Chip nach Anspruch 24 umfasst.

28. Kit nach Anspruch 27, **dadurch gekennzeichnet, dass** er auch eine komplementäre Oligonucleotidsonde der DNA-Fragmente umfasst.

29. Verwendung eines Adapters AA', wie er in einem der Ansprüche 7 bis 11 definiert ist, in Kombination mit einem Restriktionsenzym E2, wie es in Anspruch 1 definiert ist, für die Herstellung von genetischen Markern, wie sie in einem der Ansprüche 21 bis 23 definiert sind.

30. Kit zur Durchführung des Verfahrens nach einem der Ansprüche 1 bis 20, **dadurch gekennzeichnet, dass** er wenigstens einen Adapter AA', wie er in einem beliebigen der Ansprüche 7 bis 11 definiert ist, und ein Restriktionsenzym, wie es in Anspruch 1 definiert ist, umfasst.

31. Kit nach Anspruch 30, **dadurch gekennzeichnet, dass** er auch wenigstens einen Adapter BB', wie er in Anspruch 1, 16 oder 17 definiert ist, und ein Paar von Primern, wie sie in Anspruch 1 oder 18 definiert sind, umfasst.
